# EUROPEAN PATENT APPLICATION

(11) **EP 1 686 187 A1**
(43) Date of publication of application: **02.08.2006**
(21) Application number: 04818565.6
(22) Date of filing: 12.11.2004
(51) Int. Cl.: C12P 21/02, C07K 14/00, C12N 15/09

(54) **METHOD OF POSTTRANSLATIONAL MODIFICATION BY ADDING MYCROSOMAL MEMBRANE IN CELL-FREE PROTEIN SYNTHESIS**

(30) Priority: 13.11.2003 JP 2003384387
(71) Applicant: SHIMADZU CORPORATION, Kyoto-shi, Kyoto 604-8511 (JP); National University Corporation Yamaguchi University, Yamaguchi-shi, Yamaguchi 753-8511 (JP)
(72) Inventor: ENDO, Koki, Yokohama-shi, Kanagawa 236-0042 (JP); UTSUMI, Toshihiko, Yamaguchi-shi, Yamaguchi 753-0831 (JP); ITO, Masaaki, c/o Shimadzu Corporation, Kyoto-shi, Kyoto 604-8511 (JP)
(74) Representative: Pohlmann, Eckart
(86) International application number: PCT/JP2004/017219
(87) International publication number: WO 2005/047518

(57) **Abstract**

It is intended to provide a novel method for posttranslational modification in cell-free protein synthesis and a novel method for cell-free protein synthesis with the use of such posttranslational modification reaction. A method for synthesizing a protein in a cell-free system using an extract liquid for cell-free protein synthesis, **characterized in that** translation reaction is carried out in the presence of arthropod-derived microsomal membranes.

SEQUENCE LISTING

<110> SHIMADZU CORPORATION, National University Corporation Yamaguchi University

<120> Method of posttraslational modification by addition of microsome membrane in cell-free
protein synthesis

<130> G104090WO

<150> JP 2003-384387
<151> 2003-11-13

<160> 9

<210> 1
<211> 49
<212> DNA
<213> Artficial Sequence

<220>
<223> 5'UTR (EoNPV polyhedrin gene)

<400> 1
agtattgtag tcctttcgta attgtttgtg aaatctaaaa tacaccgta 49

<210> 2
<211> 702
<212> DNA
<213> homo sapience

<220>
<223> pro-TNF GLC
<400> 2
atgagcactg aaagcatgat ccgggacgtg gagctggccg aggaggcgct ccccaagaag 60
acaggggggc cccagggctc caggcggtgc ttgttcctca gcctcttctc cttcctgatc 120
gtggcaggcg ccaccacgct cttctgcctg ctgcactttg gagtgatcgg cccccagagg 180
gaagagtccc ccagggacct ctctctaatc agccctctgg cccaggcagt cagatcatct 240
tctcgaaccc cgagtgacaa gcctgtagcc catgttgtag caaaccctca agctgagggg 300
cagctccagt ggctgaaccg ccgggccaat gccctcctgg ccaatggcgt ggagctgaga 360
gataaccagc tggtggtgcc atcagagggc ctgtacctca tctactccca ggtcctcttc 420
aagggccaag gctgcccctc cacccatgtg ctcctcaccc acaccatcag ccgcatcgcc 480
gtctcctacc agaccaaggt caacctcctc tctgccatca agagcccctg ccagagggag 540

accccagagg gggctgaggc caagccctgg tatgagccca tctatctggg aggggtcttc    600
cagctggaga agggtgaccg actcagcgct gagatcaatc ggcccgacta tctcgacttt    660
gccgagtctg ggcaggtcta ctttgggatc attgccctgt ga

<210> 3
<211> 20
<212> DNA
<213> Artficial Sequence

<220>
<223> PCR primer

<400> 3
ttggatcctg caaaaagaac                20

<210> 4
<211> 21
<212> DNA
<213> Artificial Sequence

<220>
<223> PCR primer

<400> 4
gttcttggat ccctcgagaa t              21

<210> 5
<211> 21
<212> DNA
<213> Artficial Sequence

<220>
<223> PCR primer

<400> 5
cccaagctta aaaaacccct c              21

<210> 6
<211> 21
<212> DNA
<213> Artficial Sequence

<220>
<223> PCR primer

<400> 6
aaaaagcttc ccctggcgta a          21

<210> 7
<211> 22
<212> DNA
<213> Artficial Sequence

<220>
<223> His-Tag coding DNA

<400> 7
ccaccaccac caccaccact ga          22

<210> 8
<211> 26
<212> DNA
<213> Artficial Sequence

<220>
<223> PCR primer

<400> 8
cgggatccat gagcactgaa agcatg          26

<210> 9
<211> 26
<212> DNA
<213> Artficial Sequence

<220>
<223> PCR primer

<400> 9
cggaattcca gggcaatgat cccaaa          26

## Description

### TECHNICAL FIELD

The present invention relates to a cell-free protein synthesis method, and particularly to a cell-free protein synthesis method capable of carrying out posttranslational modification.

### BACKGROUND ART

Technology for mass production of proteins is indispensable for structural or functional analysis of proteins. However, it is practically difficult for expression systems using various living cells such as *Escherichia coli* to synthesize proteins interfering with the growth of the cells. For this reason, only limited kinds of proteins have been synthesized for analysis. On the other hand, cell-free systems for protein synthesis are specialized systems for artificial protein synthesis containing only components necessary for protein synthesis and, therefore, are expected to solve the problems that the expression systems using living cells are facing.

Meanwhile, proteome analysis is proceeding to comprehensively identify the structures and functions of all the proteins present in a living organism with the progress of genome analysis. In a living body, proteins are synthesized through translation on free ribosomes in the cytoplasm, and during or after translation, they are subjected to posttranslational modifications such as processing by proteases and modification of specific amino acid residues with few exceptions. These posttranslational modifications are often directly involved in functional expression of proteins and control thereof, and therefore analysis of posttranslational modifications is indispensable to identify functions of proteins.

As cell-free protein synthesis methods, methods using *Escherichia coli* lysate, wheat germ extract, or rabbit reticulocyte lysate are generally known. Further, as a protein synthesis system capable of carrying out major posttranslational modifications occurring in higher animal and plant cells, a system containing rabbit reticulocyte lysate and canine pancreatic microsomal membranes has been already known (as for canine pancreatic microsomal membranes, see for example, Walter, P. and Blobel, G., Method Enzymology (USA), vol. 96, pp. 84 - 93, 1983; Bulleid, N. J. et al., The Biochemical journal (USA), vol. 268, pp. 777 - 781, 1990; and Paradis, G. et al., Biochemistry and cell biology (Canada), vol. 65, pp. 921 - 924, 1987). However, such a system is not suitable for comprehensive synthesis of various proteins because canine pancreatic microsomal membranes are very expensive. In order to solve such a problem, a system obtained by adding fractions of endoplasmic reticulum, Golgi apparatus, and cell membrane separately prepared from Chinese hamster ovary cells (CHO cells) to rabbit reticulocyte lysate has been developed (see, for example, Japanese Patent Laid-Open Publication No. 2002-238595). However, much effort has been expended on attempting to prepare posttranslational modification machinery. A method for preparing an insect-derived extract liquid having posttranslational modification activity, the method comprising the step of applying pressure to cells in an atmosphere of an inert gas by using a special apparatus, has been developed (see, for example, Japanese Patent Laid-Open Publication No. 2000-325076). However, an extract liquid obtained by the method is not suitable for practical use in that whether or not modification occurs depends on the combination of 5'-UTR (untranslated sequence) of mRNA and the signal sequence of a target gene. In addition, this method requires a special apparatus, and is therefore lacking in versatility.

Under the circumstances, there has been demand for a versatile method for carrying out posttranslational modification in cell-free protein synthesis applicable to various extract liquids.

### DISCLOSURE OF THE INVENTION

### Objects of the Invention

The present invention has been made to solve the above-mentioned problems, and an object thereof is to provide a novel method for posttranslational modification in cell-free protein synthesis and a novel method for cell-free protein synthesis with the use of such posttranslational modification reaction.

### Summary of the Invention

In order to achieve the above-mentioned object, the present inventors have intensively investigated, and as a result they have completed the present invention.

Accordingly, the present invention provides the following.
(1) A method for synthesizing a protein in a cell-free system using an extract liquid for cell-free protein synthesis, the method comprising translation reaction in the presence of arthropod-derived microsomal membranes.
(2) The method according to the above (1), wherein in the translation reaction, the ratio of the concentration of mRNA (µg/mL) to the concentration of the arthropod-derived microsomal membranes (A260) is 1 : 0.1 - 5.
(3) The method according to the above (2), wherein the ratio is 1 : 0.3 - 2.3.
(4) The method according to any one of the above (1) - (3), wherein the arthropod-derived microsomal membranes are extracted from insect tissue.
(5) The method according to the above (4), wherein the insect tissue is a tissue of *Bombyx mori L.*
(6) The method according to the above (5), wherein the tissue of *Bombyx mori* L. is a fat body.
(7) The method according to any one of the above (1) - (3), wherein the arthropod-derived microsomal membranes are extracted from cultured insect cells.
(8) The method according to the above (7), wherein the cultured insect cells are derived from an ovum of *Trichoplusia ni* or from an ovary cell of *Spodoptera frugiperda.*
(9) The method according to any one of the above (1) - (3), wherein the extract liquid for cell-free protein synthesis comprises an arthropod-derived extract.
(10) The method according to the above (9), wherein the arthropod-derived extract is extracted from insect tissue.
(11) The method according to the above (10), wherein the insect tissue is a tissue of *Bombyx mori L.*
(12) The method according to the above (11), wherein the tissue of *Bombyx mori* L. comprises at least a posterior silk gland of *Bombyx mori* L. larva.
(13) The method according to the above (9), wherein the arthropod-derived extract is extracted from cultured insect cells.
(14) The method according to the above (13), wherein the cultured insect cells are derived from an ovum of *Trichoplusia ni* or from an ovary cell of *Spodoptera frugiperda.*
(15) The method according to any one of the above (1) - (3), wherein the extract liquid for cell-free protein synthesis comprises an extract derived from wheat germ.
(16) The method according to any one of the above (1) - (3), wherein the extract liquid for cell-free protein synthesis comprises an extract derived from cultured mammalian cells.
(17) The method according to any one of the above (1) - (3), wherein the extract liquid for cell-free protein synthesis comprises an extract derived from rabbit reticulocyte.
(18) The method according to any one of the above (1) - (3), wherein the extract liquid for cell-free protein synthesis comprises an extract derived from *Escherichia coli.*
(19) The method according to any one of the above (1) - (3), wherein the extract liquid for cell-free protein synthesis comprises an extract derived from yeast.
(20) A method for posttranslational modification of protein in cell-free protein synthesis using an extract liquid for cell-free protein synthesis, the method comprising translation reaction in the presence of arthropod-derived microsomal membranes.
(21) The method according to the above (20), wherein in the translation reaction, the ratio of the concentration of mRNA (µg/mL) to the concentration of the arthropod-derived microsomal membranes (A260) is 1 : 0.1 - 5.
(22) The method according to the above (21), wherein the ratio is 1 : 0.3 - 2.3.
(23) The method according to any one of the above (20) - (22), wherein the arthropod-derived microsomal membranes are extracted from insect tissue.
(24) The method according to the above (23), wherein the insect tissue is a tissue of *Bombyx mori L.*
(25) The method according to the above (24), wherein the tissue of *Bombyx mori* L. is a fat body.
(26) The method according to any one of the above (20) - (22), wherein the arthropod-derived microsomal membranes are extracted from cultured insect cells.
(27) The method according to the above (26), wherein the cultured insect cells are derived from an ovum of *Trichoplusia ni* or from an ovary cell of *Spodoptera frugiperda.*
(28) The method according to any one of the above (20) - (22), wherein the extract liquid for cell-free protein synthesis comprises an arthropod-derived extract.
(29) The method according to the above (28), wherein the arthropod-derived extract is extracted from insect tissue.
(30) The method according to the above (29), wherein the insect tissue is a tissue of *Bombyx mori* L.
(31) The method according to the above (30), wherein the tissue of *Bombyx mori* L. comprises at least a posterior silk gland of *Bombyx mori* L. larva.
(32) The method according to the above (28), wherein the arthropod-derived extract is extracted from cultured insect cells.
(33) The method according to the above (32), wherein the cultured insect cells are derived from an ovum of *Trichoplusia ni* or from an ovary of *Spodoptera frugiperda.*
(34) The method according to any one of the above (20) - (22), wherein the extract liquid for cell-free protein synthesis comprises an extract derived from wheat germ.
(35) The method according to any one of the above (20) - (22), wherein the extract liquid for cell-free protein synthesis comprises an extract derived from cultured mammalian cells.
(36) The method according to any one of the above (20) - (22), wherein the extract liquid for cell-free protein synthesis comprises an extract derived from rabbit reticulocyte.
(37) The method according to any one of the above (20) - (22), wherein the extract liquid for cell-free protein synthesis comprises an extract derived from *Escherichia coli.*
(38) The method according to any one of the above (20) - (22), wherein the extract liquid for cell-free protein synthesis comprises an extract derived from yeast.
(39) The method according to any one of the above (20) - (22), wherein the posttranslational modification of protein is N-glycosylation and/or signal sequence cleavage.
(40) An N-glycosylated protein which is obtained by the protein synthesis method according to any one of the above (1) - (3).
(41) A protein having a cleaved signal sequence, which is obtained by the protein synthesis method according to any one of the above (1) - (3).

According to the present invention, it is possible to provide a cell-free protein synthesis method capable of carrying out posttranslational modification of protein such as signal peptide cleavage or N-glycosylation.

### BRIEF DESCRIPTION OF THE DRAWINGS

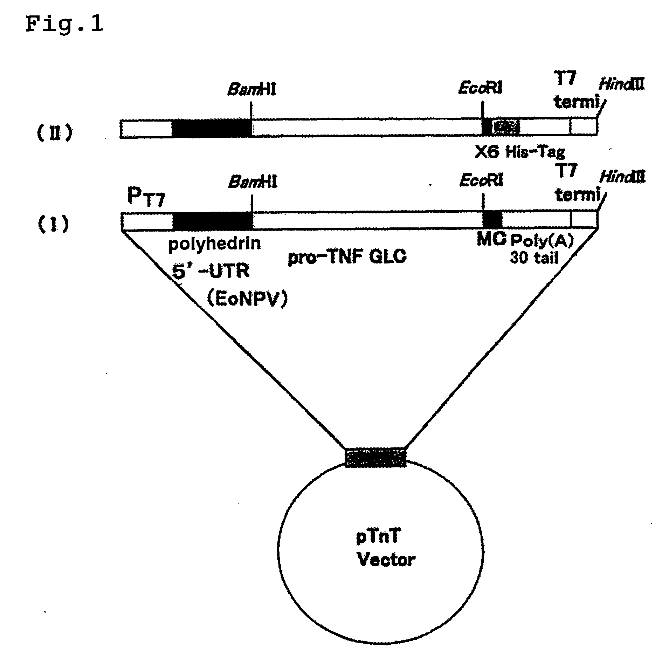

Fig. 1 is a schematic viewwhich shows a constructedplasmid (plasmid-I (I) for *in vitro* transcription of pro-TNF-GLC gene and plasmid-II (II) for *in vitro* transcription of pro-TNF-GLC gene).

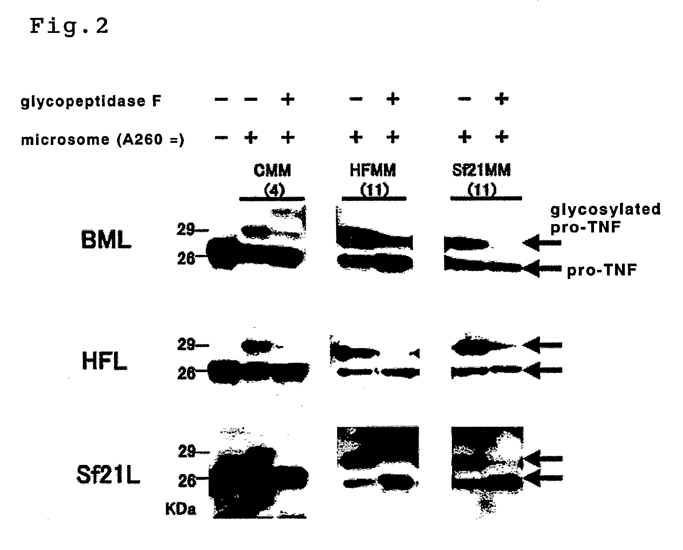

Fig. 2 shows the result of the examination carried out to detect N-glycosylation of proteins synthesized in the presence or absence of different microsomal membranes (canine pancreas-derived microsomal membranes (CMM), High Five-derived microsomal membranes (HFMM), or Sf21-derived microsomal membranes (Sf21MM)) with the use of different extract liquids for cell-free protein synthesis containing an arthropod-derived extract *(Bombyx mori* L.-derived extract (BML), High Five-derived extract (HFL), or Sf21-derived extract (Sf21L)).

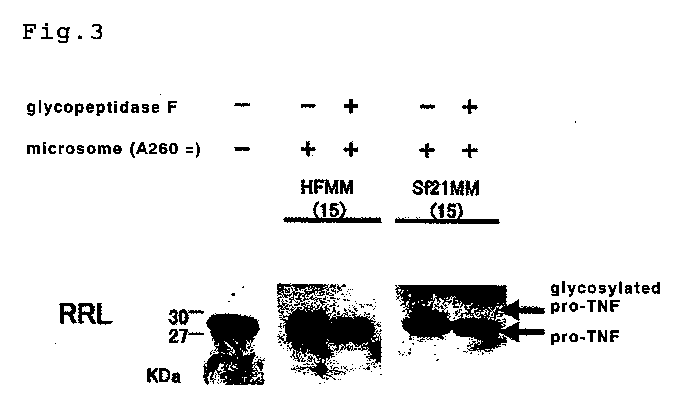

Fig. 3 shows the result of the examination carried out to detect glycosylation of proteins synthesized in the presence or absence of different microsomal membranes (High Five-derived microsomal membranes (HFMM) or Sf21-derived microsomal membranes (Sf21MM)) with the use of an extract liquid for cell-free protein synthesis containing rabbit reticulocyte lysate.

### MODES FOR CARRYING OUT THE INVENTION

Hereinafter, the present invention will be described in detail.

The present invention provides a method for synthesizing a protein in a cell-free system with the use of an extract liquid for cell-free protein synthesis, characterized in that translation reaction is carried out in the presence of arthropod-derived microsomal membranes.

Generally, cell-free protein synthesis is carried out by adding a transcription template or a translation template to a reaction liquid for cell-free protein synthesis containing an extract of biological origin containing ribosomes or the like as a translator. The translation template may be mRNA obtained by transcription from a DNA template.

An extract contained in a reaction liquid for cell-free protein synthesis to be used in the present invention is not particularly limited, as long as it allows a translation template to be translated into a protein encoded by the template. For example, conventionally known extracts or extract liquids derived from *Escherichia coli*, germs of plant seeds such as spinach and grass plants (e.g., wheat, barley, rice, corn), rabbit reticulocyte, and the like can be used without any particular limitation. Such conventionally known extracts or extract liquids are either commercially available or prepared according to methods well known per se. Specifically, extract liquids derived from *Escherichia coli,* wheat germ, and rabbit reticulocyte can be prepared according to methods described in "Biochemical Experimentation Methods 43 - Method for studying Gene Expression (Japan Scientific Societies Press)". Examples of commercially available cell extract liquids for protein synthesis include RTS100 E. coli HY Kit (manufactured by Roche Diagnostics) derived from *Escherichia coli,* Rabbit Reticulocyte Lysate System, Nuclease Treated (manufactured by Promega) derived from rabbit reticulocyte, and PROTEIOS set (manufactured by TOYOBO) derived from wheat germ. Further, an extract liquid derived from yeast can be prepared according to a method proposed by Gasior, E. et al. (see J. Biol. Chem. , 254, 3965 - 3969, 1979), a method proposed by Hussain, I. et al. (see Gene, 46, 13 - 23, 1986), or a method proposed by the present inventors (see Japanese Patent Application No. 2003-001317).

A reaction liquid for cell-free protein synthesis in the present invention may contain either such a well-known extract or extract liquid as described above or an arthropod-derived extract that has been proposed by the present inventors.

Herein, "arthropods" belong to a phylum of Metazoa, and refer to bilateral animals with a schizocoel, that is, animals called Protostomia, and include animals belonging to Chelicerata and Mandibulata. For example, animals belonging to Insecta and Arachnida are included. Among them, arthropods belonging to Insecta and Arachnida (especially, Araneomorpha) are preferred, and arthropods belonging to Insecta are particularly preferred. Examples of arthropods belonging to Insecta include, but are not limited to, those belonging to Lepidoptera, Orthoptera, Diptera, Hymenoptera, Coleoptera, Neuroptera, and Hemiptera. Among them, arthropods belonging to Bombycidae and Noctuidae of the order Lepidoptera are preferably used.

In the present invention, such an arthropod-derived extract to be contained in a reaction liquid for cell-free protein synthesis can be extracted from any tissue of an arthropod irrespective of its stage of growth or from cultured cells derived from any tissue of an arthropod. Among them, an extract extracted from a tissue of *Bombyx mori* L. or from cultured insect cells is particularly preferred.

The "*Bombyx mori* L." means an insect of Lepidoptera (Silkmoth) belonging to Bombycidae. In its life, it goes through the stages of "egg (embryo)" (from immediately after oviposition to immediately before hatching), "larva" (from immediately after hatch to immediately before completion of formation of cocoon (the first instar laraval stage -the fifth instar laraval stage)), "pupae" (from immediately before completion of formation of cocoon to immediately before eclosion), and "imago (moth)" (from immediately after eclosion to death), and "*Bombyx mori* L." includes any stage over its lifetime. *Bombyx mori* L. in the stage of larva after hatching of the egg alternately repeats the period of eating Mulberry to grow (instar) and the period of getting ready for molting without eating (diapause). In the larva of *Bombyx mori L.,* the period of from hatching to the first molting is called the first instar larval stage, and the period of from the first molting to the second molting is called the second instar larval stage, and the larva generally gets matured after four times of molting and in the fifth instar larval stage *(Bombyx mori* L. larva in the matured state is also called a "mature larva"). The mature larva of *Bombyx mori* L. has a transparent body, expectorates a silk thread to form a cocoon for pupation. After pupae, it closes into an imago.

In a case where the reaction liquid for cell-free protein synthesis contains an extract derived from a tissue of *Bombyx mori L. , Bombyx mori* L. to be used may be in any stage of its life (egg, larva (the first instar larval stage - the fifth instar larval stage), pupae, imago). The tissue of *Bombyx mori* L. is not limited to a single tissue in a single state (e.g., only posterior silk gland of *Bombyx mori* L. larvae in the fifth instar larval stage), and may be derived from plural tissues in a single state (e.g. , posterior silk gland and fat body of *Bombyx mori* L. larvae in the fifth instar larval stage) or from a single tissue in plural states (e.g., posterior silk gland of *Bombyx mori* L. larvae in the third instar larval stage, the fourth instar larval stage, and the fifth instar larval stage). Needless to say, the tissue of *Bombyx mori* L. may be derived from plural tissues in plural states. It is to be noted that the tissue of *Bombyx mori* L. to be used does not need to be the entirety thereof (e.g., entire posterior silk gland).

The "silk gland" of *Bombyx mori* L. tissue refers to a pair of tubular exocrine glands which continue from spinneret located on the tip of labium on the head to culdesac on both sides of the body of *Bombyx mori* L. larva, and is roughly divided into an anterior silk gland, a middle silk gland and a posterior silk gland. The posterior silk gland secretes fibroin that constitutes the center portion of silk. The middle silk gland secretes sericin. The fibroin is accumulated in the middle silk gland and coated with sericin on the outer periphery, and forms a gel silk substance. This silk substance is discharged from spinneret through anterior silk gland and solidified to give silk.

The "fat body" of *Bombyx mori* L. tissue is distributed in any part of the body of *Bombyx mori* L. larva and is a white soft and flat band, belt or leaf tissue. Since fat body stores nutrition and energy source like human liver, the cell contains various substances related to the metabolism such as fat drop, protein, glycogen and the like.

The "embryo" means a tissue of *Bombyx mori* L. in the state of egg.

Further, in a case where the reaction liquid for cell-free protein synthesis contains an extract derived from a tissue of *Bombyx mori* L. , the extract is preferably derived from at least one selected from the larval silk gland of *Bombyx mori* L., the larval fat body of *Bombyx mori* L., and the embryo of *Bombyx mori* L. When an extract liquid is prepared from the larval silk gland (especially, larval posterior silk gland) of *Bombyx mori* L., there is a particularly excellent advantage in that a large amount of protein can be synthesized in a short period of time. When an extract liquid is prepared from the larval fat body of *Bombyx mori* L., there is an advantage in that the extract liquid can be easily prepared because the fat body tissue is soft and can be mashed in a short period of time. When an extract liquid is prepared from the embryo of *Bombyx mori* L., there is an advantage in that the extract liquid can be easily prepared because, unlike the other tissues, embryo is a single individual and a step of isolation is not necessary.

In a case where an extract to be contained in the reaction liquid for cell-free protein synthesis is extracted from *Bombyx mori* L. , any of *Bombyx mori* L. larvae in the first instar larval stage - the fifth instar larval stage can be used, but *Bombyx mori* L. larvae in the fifth instar larval stage are preferably used. This is because tissues of a *Bombyx mori* L. larva mature toward the stage of cocoon formation, and tissues of a *Bombyx mori* L. larva in the fifth instar larval stage are the most mature among those in the first instar larval stage - the fifth instar larval stage. Therefore, the same amount of an extract can be obtained from a smaller number of *Bombyx mori* L. larvae. Particularly, in a case where the reaction liquid for cell-free protein synthesis contains an extract derived from the silk gland or fat body of *Bombyx mori* L. larvae in the fifth instar larval stage(preferably, posterior silk gland of *Bombyx mori* L. larvae in the fifth instar larval stage, more preferably posterior silk gland of *Bombyx mori* L. larvae at day 3 - day 7 in the fifth instar larval stage), there is an advantage in that a larger amount of protein can be synthesized in a short period of time as compared to a case where *Bombyx mori* L. larvae in other larval stages are used.

As described above, the arthropod-derived extract to be contained in the reaction liquid for cell-free protein synthesis may be one obtained from well-known arthropod-derived cultured cells. Preferred examples of such cultured cells include those derived from insects (hereinafter, also simply referred to as "cultured insect cell") of Lepidoptera, Hemiptera and the like, because many culture cell lines thereof have been established, and, unlike many cultured mammalian cells, these insect cells do not need to be cultured in an atmosphere of carbon dioxide, and these insect cells can be cultured in a serum-free medium. The cultured insect cells may be derived from any tissue, and, for example, blood cells, gonad-derived cells, fat body-derived cells, embryo-derived cells, hatchling-derived cells, and the like can be used without any particular limitation. Among them, gonad-derived cells that are considered to have high protein production ability are preferably used. Particularly preferred examples of cultured insect cells include High Five cells (manufactured by Invitrogen) that are derived from the ovum of *Trichoplusia ni;* and Sf 21 cells (manufactured by Invitrogen) that are derived from the ovary cell of *Spodoptera frugiperda*, because they have high protein synthesis ability in cell-system and can be cultured in a serum-free medium.

The above-mentioned reaction liquid for cell-free protein synthesis containing an arthropod-derived extract can be obtained by preparing an extract liquid (an extract liquid for cell-free protein synthesis, extract liquid = solution for extraction + extract) obtained by carrying out extraction from arthropod-derived tissue or arthropod-derived cultured cells by the use of a conventional solution for extraction having an appropriate composition, and adding components necessary for translation reaction (which will be described later) and , if necessary, adding components necessary for translation reaction and transcription reaction.

The solution for extraction to be used for carrying out extraction from arthropods is not particularly limited, but preferably contains at least a protease inhibitor. When the solution for extraction contains a protease inhibitor, there is an advantage in that the activity of protease contained in an arthropod-derived extract is inhibited, thereby preventing undesired decomposition of active protein contained in the extract due to the protease and, in turn, enabling the arthropod-derived extract to effectively exhibit its protein synthesis ability. The above-mentioned protease inhibitor is not particularly limited as long as it can inhibit the activity of protease, and, for example, phenylmethanesulfonyl fluoride (hereinafter sometimes to be referred to as "PMSF"), aprotinin, bestatin, leupeptin, pepstatin A, E-64 (L-trans-epoxysuccinyl-L-leucylamido(4-guanidino)butane), ethylenediaminetetraacetic acid, phosphoramidon and the like can be used. Since an extract derived from arthropods often contains serine protease, the use of PMSF, which works as an inhibitor having high specificity to serine protease, is preferable among those mentioned above. It is possible to use not only one kind of protease inhibitor but also a mixture (protease inhibitor cocktail) of several kinds of protease inhibitors.

The content of the protease inhibitor in the solution for extraction is free of any particular limitation, but it is preferably 1 µM - 50 mM, more preferably 0.01 mM - 5 mM, because decomposition of the enzyme necessary for cell-free protein synthesis can be preferably inhibited. This is because the decomposition activity of protease often cannot be suppressed sufficiently when the protease inhibitor content is less than 1 µM, and the protein synthesis reaction tends to be inhibited when the protease inhibitor content exceeds 50 mM.

The solution for extraction to be used for the present invention preferably contains, in addition to the above-mentioned protease inhibitor, at least a potassium salt, a magnesium salt, DTT and a buffer.

The above-mentioned potassium salt can be used in a general form, such as potassium acetate, potassium carbonate, potassium hydrogen carbonate, potassium chloride, dipotassium hydrogen phosphate, dipotassium hydrogen citrate, potassium sulfate, potassium dihydrogen phosphate, potassium iodide, potassium phthalate and the like, with preference given to potassium acetate. Potassium salt acts as a cofactor in the protein synthesis reaction.

The content of the potassium salt in the solution for extraction is free of any particular limitation, but from the aspect of preservation stability, it is preferably 10 mM - 500 mM, more preferably 50 mM - 300 mM, in the case of a monovalent potassium salt, such as potassium acetate and the like. When the content of the potassium salt is less than 10 mM or more than 500 mM, the components essential for protein synthesis tend to become unstable.

The above-mentionedmagnesium salt can be used in a general form such as magnesium acetate, magnesium sulfate, magnesium chloride, magnesium citrate, magnesium hydrogen phosphate, magnesium iodide, magnesium lactate, magnesium nitrate, magnesium oxalate and the like, with preference given to magnesium acetate. Magnesium salt also acts as a cofactor in the protein synthesis reaction.

The content of the magnesium salt in the solution for extraction is free of any particular limitation, but from the aspect of preservation stability, it is preferably 0.1 mM - 10 mM, more preferably 0.5 mM - 5 mM, in the case of a divalent salt, such as magnesium acetate and the like. When the content of the magnesium salt is less than 0.1 mM or more than 10 mM, the components essential for protein synthesis tend to become unstable.

The above-mentioned DTT is added for prevention of oxidization, and is preferably contained in an amount of 0.1 mM - 10 mM, more preferably 0.5 mM - 5 mM, in the solution for extraction. When the content of DTT is less than 0.1 mM or more than 10 mM, the components essential for protein synthesis tend to become unstable.

The above-mentioned buffer imparts a buffer capacity to the solution for extraction, and is added for prevention of denaturation of an extract caused by a radical change in pH of an extract liquid, which is due to, for example, addition of an acidic or basic substance and the like. Such buffer is free of any particular limitation, and, for example, HEPES-KOH, Tris-HCl, acetic acid-sodium acetate, citric acid-sodium citrate, phosphoric acid, boric acid, MES, PIPES and the like can be used.

The buffer is preferably one that maintains the pH of the obtained extract liquid at 4-10, more preferably pH 6.5-8.5. When the pH of the extract liquid is less than 4 or more than 10, the components essential for the reaction of the present invention may be denatured. From this aspect, the use of HEPES - KOH (pH 6.5-8.5) is particularly preferable among the above-mentioned buffers.

While the content of the buffer in the solution for extraction is free of any particular limitation, it is preferably 5 mM - 200 mM, more preferably 10 mM - 100 mM, to maintain preferable buffer capacity. When the content of the buffer is less than 5 mM, pH tends to change radically due to the addition of an acidic or basic substance, which in turn may cause denaturation of the extract, and when the content of the buffer exceeds 200 mM, the salt concentration becomes too high and the components essential for protein synthesis tend to become unstable.

In a case where the arthropod-derived extract is extracted from cultured insect cells, it is preferred that the solution for extraction further contains calcium chloride and glycerol in addition to the above-mentioned components. By using such a solution for extraction, it is possible to obtain an extract liquid of cultured insect cells having more improved protein synthesis ability.

In this case, the content of calcium chloride is not particularly limited, but is preferably 0.1 mM - 10 mM, more preferably 0.5 mM - 5 mM, from the viewpoint of effectively improving protein synthesis ability. Further, the content of glycerol to be added is not also particularly limited, but it is preferably added in a proportion of 5 (v/v)% - 80 (v/v)%, more preferably in a proportion of 10 (v/v)% - 50 (v/v)%, from the viewpoint of effectively improving protein synthesis ability.

A method for preparing an extract liquid from arthropod-derived tissue or arthropod-derived cultured cells is not particularly limited, and a conventional method can be used.

For example, in a case where as an arthropod-derived extract, an extract extracted from a tissue of *Bombyx mori* L. or cultured insect cells is used, an extract liquid is preferably prepared by an extraction method proposed by the present inventors with the use of a solution for extraction having the above-mentioned composition.

Hereinafter, a method for preparing an extract liquid containing an extract derived from a tissue of *Bombyx mori* L. and a method for preparing an extract liquid containing an extract derived from cultured insect cells will be described in detail.

### [A] Method for Preparing Extract Liquid Containing Extract Derived from Tissue of Bombyx mori L.

First, according to a conventional method, a desired tissue is isolated *from Bombyxmori* L using tools such as scissors, tweezers, and a scalpel. It is to be noted that the amount of the tissue to be used for extraction (which will be described later) is not particularly limited, but is usually in the range of 1 - 100 g.

Then, the isolated tissue is frozen with, for example, liquid nitrogen, and is mashed in a mortar frozen at -80°C. The above-mentioned solution for extraction is added to the mashed tissue to carry out extraction.

Alternatively, after the addition of the solution for extraction, a mixture of the tissue and the solution for extraction may be frozen. In this case, the frozen mixture is stirred with a spatula until it is melted into a sherbet state (specifically, until it is melted into a wet, crunchy, and yellow ice state). Thereafter, the mixture is again frozen completely with liquid nitrogen, and then the frozen mixture is stirred with a spatula until it is melted into a sherbet state (specifically, until it is melted into a wet, crunchy, and yellow ice state). By doing so, it is possible to effectively extract components necessary for protein synthesis and to stabilize these components.

In this way, a liquid containing an extract from a tissue of *Bombyx mori* L. is obtained.

Next, the liquid obtained by the extraction treatment described above is centrifuged under the conditions generally used in this field (i.e., 10,000 xg - 50,000 xg, 0 - 10°C, 10 - 60 minutes). Supernatant obtained by carrying out centrifugal separation once (hereinafter, referred to as "supernatant A1") can be used as it is as an extract liquid. Alternatively, the supernatant A1 may be again centrifuged under the same conditions described above. In this case, the obtained supernatant (hereinafter, referred to as "supernatant A2") can be used as an extract liquid.

Alternatively, a precipitation obtained by carrying out centrifugal separation for the first time may be further subjected to extraction using the above-mentioned solution for extraction. In this case, the obtained liquid is centrifuged under the same conditions described above to obtain supernatant (hereinafter, referred to as "supernatant A3"), and then the supernatant A1 and the supernatant A3 may be mixed together to prepare an extract liquid. By using an extract liquid prepared by mixing the supernatant A1 and the supernatant A3, it is possible to further improve the efficiency of protein synthesis as compared to a case where the supernatant A1 or the supernatant A3 is used singly as an extract liquid. Alternatively, the supernatant A2 may be mixed with the supernatant A3 to prepare an extract liquid. In this case, the above-mentioned effect is further improved. Needless to say, the supernatants A1 - A3 may be mixed together to prepare an extract liquid.

In above-described case, the mixing volume ratio between the supernatant A1 and/or the supernatant A2 (in a case where both of the supernatant A1 and the supernatant A2 are used, the total volume of them) and the supernatant A3 in a mixture (that is, in an extract liquid) is not particularly limited, but is preferably 10 : 90 - 90 : 10, more preferably 20 : 80 - 80 : 20, from the viewpoint of efficiency of protein synthesis.

Alternatively, each of the extract liquids prepared in such a manner described above may be subjected to gel filtration. In this case, fractions having the highest absorbance at 280 nm and an absorbance in the vicinity of the highest absorbance at 280 nm may be collected from filtrate obtained by gel filtration to prepare an extract liquid. However, an extract liquid is preferably prepared without carrying out such gel filtration and fractionation, from the viewpoint of efficiency of protein synthesis.

As described above, in a case where gel filtration is carried out and fractions having the highest absorbance at 280 nm and an absorbance in the vicinity of the highest absorbance at 280 nm are collected from filtrate obtained by gel filtration, the following steps may be concretely performed.

According to a conventional method, a column, for example, a desalting column PD-10 (manufactured by Amersham Biosciences) is equilibrated using a buffer solution for gel filtration, and then a sample is fed to the column and is eluted with the above-mentioned solution for extraction. The buffer solution for gel filtration is preferably obtained by adding glycerol to the above-mentioned solution for extraction. Glycerol is usually added to the solution for extraction in a proportion of 5 (v/v)% - 40 (v/v)%, preferably in a proportion of 20 (v/v)%. The filtrate obtained by gel filtration is fractionated into 0.1 mL - 1 mL fractions as in the case of general gel filtration, but is preferably fractionated into 0.4 mL - 0.6 mL fractions from the viewpoint of efficiently obtaining a fraction (s) having high protein synthesis ability.

Then, a fraction(s) having an absorbance at 280 nm of 10 or higher is (are) collected from the filtrate obtained by gel filtration. In this step, the absorbance of each of the fractions is measured at 280 nm with an instrument, for example, Ultrospec3300pro (manufactured by Amersham Biosciences), and fractions having the highest absorbance and an absorbance in the vicinity of the highest absorbance are collected and used as an extract liquid.

### [B] Method for Preparing Extract Liquid Containing Extract Derived from Cultured Insect Cells

In a case where an extract liquid is prepared from cultured insect cells, a method proposed by the present inventors is preferably used. Specifically, a method which comprises at least a step of rapidly freezing cultured insect cells suspended in a solution for extraction is preferably used. Herein, the phrase "rapidly freezing" means that cultured insect cells are frozen in 10 seconds or shorter, preferably in 2 seconds or shorter, after the cultured insect cells are subjected to freezing treatment. The cultured insect cells are generally rapidly frozen at -80°C or lower, preferably -150°C or lower. The rapid freezing of cultured insect cells can be realized by, for example, using an inert gas such as liquid nitrogen or liquid helium. Among these inert gases, liquid nitrogen is preferably used because it is easily available and economical.

By carrying out extraction from cultured insect cells in such a manner described above, cells can be ruptured under mild conditions, and therefore components essential for cell-free protein synthesis can be taken out from the cells without damage. As a result, it is possible to easily prepare an extract liquid for cell-free protein synthesis having higher ability to synthesize protein than that prepared by a conventional method. In addition, it is also possible to prevent contamination of RNase and the like from tools etc. Further, there is no possibility of incorporation of a substance inhibiting translation reaction, which is of concern in a case where a cell rupture method using a reagent such as a surfactant is employed.

The extract liquid preparation method proposed by the present inventors is not particularly limited in respect of other steps as long as it comprises at least the step of rapid freezing as described above. For example, cultured insect cells may be ruptured and subjected to extraction by various methods conventionally used for obtaining an extract liquid for cell-free protein synthesis from *Escherichia coli,* wheat germ, or the like, such as a method comprising mashing in a mortar with a pestle, a method using a Dounce homogenizer, a method using glass beads, and the like. Among them, the cultured insect cells rapidly frozen are preferably thawed and then centrifuged to rupture the cultured insect cells.

In this case, the cultured insect cells rapidly frozen can be thawed by placing in a water bath or an ice-water bath at -10 - 20°C or by being left standing at room temperature (25°C). However, the cultured insect cells rapidly frozen are preferably thawed by placing in a water bath or an ice-water bath at 0 - 20°C (particularly preferably at 4 - 10°C) to prevent the deactivation of components essential for protein synthesis and to properly prevent the degradation of protein synthesis ability. The thawed cultured insect cells are centrifuged under the conditions generally used in this field (i.e., 10,000 xg - 50,000 xg, 0 - 10°C, 10 - 60 minutes). Supernatant obtained by centrifugal separation contains a target extract from the cultured insect cells.

After the cell rupture, the supernatant obtained by the above-mentioned centrifugal separation (hereinafter, referred to as "supernatant B1") can be used as it is as an extract liquid. Alternatively, the supernatant B1 may be further centrifuged (10,000 xg - 100,000 xg, 0 - 10°C, 10 - 120 minutes). In this case, the obtained supernatant (hereinafter, referred to as "supernatant B2") can be used as an extract liquid. Further, the supernatant B1 or the supernatant B2 may be subjected to gel filtration. In this case, a fraction(s) having an absorbance at 280 nm of 10 or higher (that is, a fraction(s) having a high absorbance) is (are) collected from filtrate obtained by gel filtration to prepare an extract liquid. In a case where the supernatant B1 or the supernatant B2 is subjected to gel filtration, the following steps are concretely performed.

In a case where the supernatant B1 or the supernatant B2 is subjected to gel filtration, according to a conventional method, a column for gel filtration, preferably, a desalting column PD-10 (manufactured by Amersham Biosciences) is equilibrated using a buffer solution for gel filtration, and then a sample is fed to the column and is eluted with the buffer solution for gel filtration. As the buffer solution for gel filtration, conventionally known buffer solutions for gel filtration having appropriate composition can be used without any particular limitation. For example, a buffer solution for gel filtration containing 10 mM - 100 mM of HEPES-KOH (pH 6.5 - 8.5), 50 mM - 300 mM of potassium acetate, 0.5 mM - 5 mM of magnesium acetate, 0.5 mM - 5 mM of DTT, and 0.01 mM - 5 mM of PMSF can be used. The filtrate obtained by gel filtration is fractionated into 0.1 mL - 1 mL fractions as in the case of general gel filtration, but is preferably fractionated into 0.4 mL - 0.6 mL fractions from the viewpoint of efficiently obtaining a fraction(s) having high protein synthesis ability.

Then, the absorbance of each of the fractions is measured at 280 nm with an instrument, for example, Ultrospec3300pro (manufactured by Amersham Biosciences), and a fraction(s) having an absorbance at 280 nm of 30 or higher (that is, a fraction (s) having a high absorbance) is (are) collected from the filtrate obtained by gel filtration to prepare an extract liquid.

Further, the obtained fraction(s) having a high absorbance may be further centrifuged. In this case, the obtained supernatant (hereinafter, referred to as "supernatant B3") can be used as an extract liquid. The centrifugal separation after gel filtration is preferably carried out at 30,000 xg - 100,000 xg at 0 - 10°C for 10 - 60 minutes, from the viewpoint of removal of insoluble components inhibiting translation reaction.

It is to be noted that cultured insect cells to be subjected to the preparation method used in the present invention are preferably washed in advance, before they are rapidly frozen as described above, with a washing solution having the same composition as that of the above-mentioned solution for extraction suitably used for cultured insect cells except that a protease inhibitor and glycerol are omitted, for the purpose of preventing a culture medium from being brought in a reaction liquid for translation. Cultured insect cells are washed by adding the washing solution to the cultured insect cells and subjecting the mixture to centrifugal separation (e.g., 700 xg, 10 min, 4°C). The amount of the washing solution to be used for washing is preferably 5 mL - 100 mL, more preferably 10 mL - 50 mL, per gram of wet cultured insect cells for completely removing a culture medium. The frequency of washing is preferably 1 - 5 times, more preferably 2 - 4 times.

The amount of an arthropod-derived extract contained in the extract liquid to be used in the present invention is not particularly limited, but is preferably 1 mg/mL - 200 mg/mL, more preferably 10 mg/mL - 100 mg/mL, in terms of protein concentration. If the arthropod-derived extract content is less than 1 mg/mL in terms of protein concentration, there is a fear that the concentrations of components essential for cell-free protein synthesis are decreased so that a protein is not sufficiently synthesized. On the other hand, if the arthropod-derived extract content exceeds 200 mg/mL in terms of protein concentration, there is a fear that the extract liquid itself has high viscosity and becomes difficult to handle.

An extract liquid containing an arthropod-derived extract in an amount within the above range can be prepared by measuring the protein concentration of the extract liquid according to a method generally used in this field. For example, in a case where BCA Protein assay Kit (manufactured by PIERCE) is used, 0.1 mL of a sample is added to 2 mL of a reaction reagent to obtain a mixture, the mixture is subjected to reaction at 37°C for 30 minutes, and the absorbance of the mixture is measured at 562 nm using a spectrophotometer (e.g., Ultrospec3300pro manufactured by Amersham Biosciences). In this case, bovine serum albumin (BSA) is usually used as a control.

In the cell-free protein synthesis method, a reaction liquid for translation system or a reaction liquid for transcription/translation system is prepared using, for example, the extract liquid prepared in such a manner described above. In either case of a reaction liquid for translation system or a reaction liquid for transcription/translation system, the reaction liquid is preferably prepared in such a manner that the extract liquid is contained in a proportion of 10 (v/v)% - 80 (v/v)%, particularly preferably in a proportion of 30 (v/v)% - 60 (v/v)%. That is, the reaction liquid is preferably prepared in such a manner that the amount of an arthropod-derived extract contained in the entire reaction liquid is 0.1 mg/mL - 160 mg/mL, more preferably 3 mg/mL - 60 mg/mL, in terms of protein concentration. If the arthropod-derived extract content is less than 0.1 mg/mL or exceeds 160 mg/mL in terms of protein concentration, the rate of protein synthesis tends to be lower.

Hereinafter, (1) a reaction liquid for translation system and (2) a reaction liquid for transcription/translation system will be described by taking, as an example, a case where an extract liquid containing an arthropod-derived extract is used. In a case where an extract liquid containing an extract other than an arthropod-derived extract is used, necessary reagents and reaction conditions are appropriately selected depending on the source of the extract.

### (1) Reaction Liquid for Translation System

A reaction liquid for translation system preferably contains, as components other than the arthropod-derived extract liquid, at least a translation template, potassium salt, magnesium salt, DTT, adenosine triphosphate, guanosine triphophate, creatine phosphate, creatine kinase, amino acid component, RNase inhibitor, tRNA, and buffer. The reaction liquid for translation system further contains microsomal membranes, specifically arthropod-derived microsomal membranes (which will be described later) for carrying out posttranslational modification of protein, that is, for achieving the object of the present invention. By carrying out translation reaction using such a reaction liquid for translation system, it is possible to synthesize a large amount of protein in a short period of time.

The number of nucleotides of the translation template (mRNA) contained in the reaction liquid for translation system is not particularly limited, and all the mRNAs do not need to have the same number of nucleotides as long as a target protein can be synthesized. In addition, plural nucleotides of each of the mRNAs may be deleted, substituted, inserted or added as long as the sequences thereof are homologous to the extent that a target protein can be synthesized. The mRNA can be prepared by transcription of a DNA template (which is prepared by, for example, the following method) according to an appropriate method conventionally known, but is preferably prepared by transcribing a DNA template by *in vitro* transcription well known per se. *In vitro* transcription can be carried out using, forexample, RiboMax Large Scale RNA production System-T7 (manufactured by Promega). The mRNA prepared by transcription is purified and isolated by a method well known per se, and as will be described later, it is used as a translation template for cell-free protein synthesis in the reaction liquid for translation system.

The translation template is preferably contained in the reaction liquid for translation system in a proportion of 1 µg/mL - 2000 µg/mL, more preferably in a proportion of 10 µg/mL - 1000 µg/mL, from the viewpoint of the rate of protein synthesis. If the amount of the mRNA contained in the reaction liquid for translation system is less than 1 µg/mL or exceeds 2000 µg/mL, the rate of protein synthesis tends to be lower.

The concentration of the arthropod-derived microsomal membranes in the reaction liquid for translation system is, in terms of absorbance at 260 nm (hereinafter, referred to as "A260"), 1 - 50 (A260=1 - 50), preferably 2 - 15 (A260=2 - 15), from the viewpoint of efficiency of posttranslational modification of protein. If the concentration of the microsomal membranes is less than 1 in terms of A260, posttranslational modification tends to be insufficiently carried out. On the other hand, if the concentration of the microsomal membranes exceeds 50 in terms of A260, protein synthesis itself tends to be significantly inhibited. In addition, the ratio of the mRNA concentration (µg/mL) to the arthropod-derived microsomal membrane concentration (A260) in the reaction liquid for translation system is preferably 1 : 0.1 - 5, more preferably 1 : 0.3 - 2.3, from the viewpoint of efficiency of posttranslational modification of protein.

The purity of the arthropod-derived microsomal membranes at the time of determination of the ratio between the mRNA concentration (µg/mL) and the arthropod-derived microsomal membrane concentration (A260) is usually A260/A280 = 1.3 - 2.0, preferably A260/A280 = 1.4 - 1.8. It is to be noted that the fact that the ratio of the mRNA concentration (µg/mL) to the arthropod-derived microsomal membrane concentration (A260) in the reaction liquid is 1 : 0.1 - 5 (preferably 1 : 0.3 - 2.3) means that the concentration of the microsomal membranes is 0.1 - 5 (preferably 0.3 - 2.3) in terms of A260 when 1 mL of the reaction liquid contains 1 µg of mRNA.

It is preferred that the microsomal membranes exist in the reaction liquid at the time of beginning of translation, but the timing of adding the microsomal membranes may be appropriately adjusted, if necessary.

The arthropod-derived microsomal membranes are derived from insect tissue (particularly, from a tissue of *Bombyx mori* L.) or from cultured insect cells (particularly, from the ova of *Trichoplusiani*, or the ovary cells of *Spodoptera frugiperda*), and a method for preparing such arthropod-derived microsomal membranes is not particularly limited as long as the activity of the microsomal membranes is not lost. For example, such microsomal membranes can be prepared by sucrose density gradient ultracentrifugation based on a method developed by Walter, P. and Blobel, G. (see Enzymol. 96, pp. 84 - 93, 1983). Specifically, insect tissue or cultured insect cells collected by centrifugation are suspended in 1 - 10 mL of a solution for extraction of microsomal membrane (the composition of the solution for extraction can be appropriately changed according to the kind of tissue or cell to be used) per gram of insect tissue or cultured insect cells, the suspension is treated by a homogenizer (preferably by a Dounce homogenizer) to rupture cells, and then cell debris is removed by, for example, centrifugation to collect supernatant. The supernatant is subjected to sucrose density gradient ultracentrifugation to separate a microsomal membrane fraction. A detailed method for preparing arthropod-derived microsomal membranes will be described later in EXAMPLES.

Preferred examples of the potassium salt to be contained in the reaction liquid for translation system include various potassium salts mentioned above with reference to the solution for extraction. Among them, potassium acetate is preferably used. The potassium salt is preferably contained in the reaction liquid for translation system in a proportion of 10 mM - 500 mM, more preferably in a proportion of 50 mM - 150 mM, from the same point of view as described above with reference to the potassium salt contained in the solution for extraction.

Preferred examples of the magnesium salt to be contained in the reaction liquid for translation system include various magnesium salts mentioned above with reference to the solution for extraction. Among them, magnesium acetate is preferably used. The magnesium salt is preferably contained in the reaction liquid for translation system in a proportion of 0.1 mM - 10 mM, more preferably in a proportion of 0.5 mM - 3 mM, from the same point of view as described above with reference to the magnesium salt contained in the solution for extraction.

The DTT is preferably contained in the reaction liquid for translation system in a proportion of 0.1 mM - 10 mM, more preferably in a proportion of 0. 2 mM - 5 mM, from the same point of view as described above with reference to the DTT contained in the solution for extraction.

The adenosine triphosphate (hereinafter sometimes to be referred to as "ATP") is preferably contained in the reaction liquid for translation system in a proportion of 0.01 mM - 10 mM, more preferably in a proportion of 0.1 mM - 5 mM, in view of the rate of protein synthesis. When ATP is contained in a proportion of less than 0.01 mM or above 10 mM, the synthesis rate of the protein tends to become lower.

The guanosine triphosphate (hereinafter sometimes to be referred to as "GTP") preferably contained in the reaction liquid for translation system in a proportion of 0.01 mM - 10 mM, more preferably in a proportion of 0.1 mM - 5 mM, in view of the rate of protein synthesis. When GTP is contained in a proportion of less than 0.01 mM or above 10 mM, the synthesis rate of the protein tends to become lower.

The creatine phosphate in the reaction liquid for translation system is a component for continuous synthesis of protein and added for regeneration of ATP and GTP. The creatine phosphate is preferably contained in the reaction solution in a proportion of 1 mM - 200 mM, more preferably in a proportion of 10 mM - 100 mM, in view of the rate of protein synthesis. When creatine phosphate is contained in a proportion of less than 1 mM, sufficient amounts of ATP and GTP may not be regenerated easily. As a result, the rate of protein synthesis tends to become lower. When the creatine phosphate content exceeds 200 mM, it acts as an inhibitory substance and the rate of protein synthesis tends to become lower.

The creatine kinase in the reaction liquid for translation system is a component for continuous synthesis of protein and added along with creatine phosphate for regeneration of ATP and GTP. The creatine kinase is preferably contained in the reaction solution in a proportion of 1 µg/mL - 1000 µg/mL, more preferably 10 µg/mL - 500 µg/mL, in view of the rate of protein synthesis. When the creatine kinase content is less than 1 µg/mL, sufficient amount of ATP and GTP may not be regenerated easily. As a result, the rate of protein synthesis tends to become lower. When the creatine kinase content exceeds 1000 µg/mL, it acts as an inhibitory substance and the synthesis rate of the protein tends to become lower.

The amino acid component in the reaction liquid for translation system contains at least 20 kinds of amino acids, i.e., valine, methionine, glutamic acid, alanine, leucine, phenylalanine, glycine, proline, isoleucine, tryptophan, asparagine, serine, threonine, histidine, aspartic acid, tyrosine, lysine, glutamine, cystine and arginine. This amino acid includes radioisotope-labeled amino acid. If necessary, modified amino acid may be contained. The amino acid component generally contains almost the same amount of various kinds of amino acids.

In the present invention, the above-mentioned amino acid component is preferably contained in the reaction solution in a proportion of 1 µM - 1000 µM, more preferably 10 µM - 200 µM, in view of the rate of protein synthesis. When the amount of the amino acid component is less than 1 µM or above 1000 µM, the synthesis rate of the protein tends to become lower.

The RNase inhibitor in the reaction liquid for translation system is added to prevent RNase, which is derived from arthropod contaminating the extract solution, from undesirably digesting mRNA and tRNA, thereby preventing synthesis of protein, during cell-free protein synthesis. It is preferably contained in the reaction solution in a proportion of 0.1 U/µL - 100 U/µL, more preferably in a proportion of 1 U/µL - 10 U/µL. When the amount of RNase inhibitor is less than 0.1 U/µL, the degradation activity of RNase often cannot be suppressed sufficiently, and when the amount of the RNase inhibitor exceeds 100 U/µL, protein synthesis reaction tends to be inhibited.

The tRNA in the reaction liquid for translation system contains almost the same amount of each of the tRNAs corresponding to the above-mentioned 20 kinds of amino acids. In the present invention, tRNA is preferably contained in the reaction solution in a proportion of 1 µg/mL - 1000 µg/mL, more preferably in a proportion of 10 µg/mL - 500 µg/mL, in view of the rate of protein synthesis. When the amount of tRNA is less than 1 µg/mL or exceeds 1000 µg/mL, the rate of protein synthesis tends to become lower.

Preferred examples of the buffer to be contained in the reaction liquid for translation system include various buffers mentioned above with reference to the solution for extraction. Among them, HEPES-KOH (pH 6 - 8) is preferably used for the same reason as described above. The amount of the buffer to be contained in the reaction liquid for translation system is preferably 5 mM - 200 mM, more preferably 10 mM - 50 mM, from the same point of view as described above with reference to the buffer contained in the solution for extraction.

Further, the reaction liquid for translation system preferably contains glycerol. By adding glycerol to the reaction liquid for translation system, it is possible to stabilize components essential for protein synthesis in translation system. When glycerol is added to the reaction liquid for translation system, the amount of glycerol is usually 5 (v/v)% - 20(v/v)%.

Furthermore, the reaction liquid for translation system preferably contains ethylene glycol bis(2-aminoethyl ether)tetraacetic acid (hereinafter sometimes referred to as "EGTA"). When EGTA is contained, EGTA forms chelate with a metal ion in the extract liquid to inactivate ribonuclease, protease and the like. This in turn inhibits decomposition of the components essential for cell-free protein synthesis. EGTA is preferably contained in the reaction solution at 0.01 mM - 10 mM, more preferably 0.1 mM - 5mM, in view of preferable exertion of the above-mentioned decomposition inhibitory ability. When EGTA is contained in less than 0.01 mM, decomposition activity of essential components cannot be sufficiently suppressed. When it exceeds 10 mM, it tends to inhibit protein synthesis reaction.

As described above, the reaction liquid for translation system preferably contains, in addition to 30 (v/v)% - 60 (v/v)% of the extract liquid containing an arthropod-derived extract, 50 mM - 150 mM of potassium acetate, 0.5 mM - 3 mM of magnesium acetate, 0.2 mM - 5 mM of DTT, 5 (v/v)% - 20 (v/v)% of glycerol, 0.1 mM - 5 mM of ATP, 0.1 mM - 5 mM of GTP, 10 mM - 100 mM of creatine phosphate, 10 µg/mL - 500 µg/mL of creatine kinase, 10 µM - 200 µM of amino acid component, 1 U/µL - 10 U/µL of RNase inhibitor, 10 µg/mL - 500 µg/mL of tRNA, 10 µg/mL - 1000 µg/mL of translation template, mammal-derived microsomal membranes (A260 = 1 - 50 in reaction liquid for translation system, concentration ratio of mRNA (translation template) (µg/mL) to microsomal membranes (A260) is 1 : 0.1 - 5), and 10 mM - 50 mM of HEPES-KOH (pH 6 - 8).

More preferably, the reaction liquid for translation system further contains 0.1 mM - 5 mM of EGTA.

Cell-free protein synthesis using the reaction liquid for translation system (synthesis reaction in translation system) is carried out in, for example, a low temperature incubator conventionally known. The temperature for reaction is generally 10 - 40°C, preferably 20 - 30°C. If the reaction temperature is lower than 10°C, the rate of protein synthesis tends to be lower. On the other hand, if the reaction temperature exceeds 40°C, essential components tend to be denatured. The time for reaction is generally 1 - 72 hours, preferably 3 - 24 hours.

### (2) Reaction Liquid for Transcription/Translation System

A reaction liquid for transcription/translation system preferably contains, as components other than the above-mentioned extract liquid, at least transcription template, RNA polymerase, ATP, GTP, cytidine 5'-triphosphate, uridine 5'-triphosphate, creatine phosphate, creatine kinase, amino acid component, and tRNA. Further, the reaction liquid for transcription/translation system contains microsomal membranes, specifically arthropod-derived microsomal membranes so as to achieve the object of the present invention, that is , to carry out posttranslational modification of protein. By using such a reaction liquid for transcription/translation system to carry out synthesis reaction in transcription/translation system, it is possible to synthesize a large amount of protein in a short period of time.

It is to be noted that the transcription template (DNA template) is not particularly limited in nucleotide sequence and the number of nucleotides, as long as it has at least a promoter sequence and a structural gene encoding a protein. Further, the protein (including peptide) encoded by the structural gene is not particularly limited, and the transcription template may have a nucleotide sequence encoding a protein toxic to living cells, or a nucleotide sequence encoding glycoprotein. In the DNA template, the promoter sequence is generally located 5'upstream of the structural gene. Examples of such a promoter sequence include conventionally known T7 promoter sequence, SP6 promoter sequence, and T3 promoter sequence.

The DNA template to be used in the present invention preferably has a terminator sequence 3'downstream of the structural gene. Examples of such a terminator sequence include conventionally known T7 terminator sequence, SP6 terminator sequence, and T3 terminator sequence. The DNA template may have a poly A sequence 3'downstream of the structural gene, from the viewpoint of stability of synthesized mRNA.

The DNA template may be one synthesized by a method comprising at least the steps of: (1) ligating plural regions of a DNA template previously divided; and (2) amplifying ligated DNA by PCR. Herein, the number of regions which can be combined together by a series of ligation reactions and can be amplified by PCR is two. In order to further combine with another region and amplify the obtained DNA, it is necessary to again carry out a series of ligation reactions and PCR. For this reason, the DNA template is preferably divided into regions so that the number of regions becomes as small as possible. The number of regions is preferably 2 - 5, more preferably 2 - 3, particularly preferably 2. The DNA template is divided into regions in advance so that a nucleotide sequence not present in the regions of the DNA template or an overlapping nucleotide sequence part among the regions is not amplified, that is, so that all the regions are combined into a DNA template. Each of the divided regions may be DNA prepared by cutting a plasmid DNA with a restriction enzyme, or DNA amplified by PCR, or DNA synthesized by a DNA synthesizer.

First, among the divided regions of the DNA template, two regions to be adjacent to each other are ligated together. Prior to ligation, the ends of the DNAs are preferably treated so that both of the DNAs can be linked in a forward direction. Specifically, the two DNAs to be combined together are treated to have blunt ends. Alternatively, the two DNAs to be combined together may be cleaved with the same restriction enzyme. Further, the ends of one of the DNAs are preferably phosphorylated with T4 polynucleotide kinase so that ligation is efficiently carried out. The ligation reaction can be carried out using reagents and conditions generally used in this field. For example, Quick Ligation Kit (manufactured by NEB) can be used. In this case, according to the protocol, DNA, reaction buffer, and Quick T4 Ligase are mixed, and the mixture is incubated at 25°C for 5 minutes.

Next, the ligated DNA is amplified by PCR. PCR can be carried out using a conventionally known PCR machine such as a commercially available thermal cycler for PCR under conditions generally used in this field. The PCR machine to be used and conditions for PCR are not particularly limited. For example, KOD plus (manufactured by TOYOBO) can be used, according to the protocol. In this case, a sense primer and an anti-sense primer prepared based on the sequences of the ends of DNA to be amplified are used as primers. By using such primers, it is possible to amplify only a desired DNA by PCR among various DNAs obtained by ligation.

In a case where the DNA template has been divided into three or more regions, the DNA amplified by PCR and another DNA to be combined with the amplified DNA are further subjected to ligation and PCR to prepare a DNA template.

It is preferred that the DNA template in the present invention further contains a sequence having the activity to promote translation reaction (hereinafter, also referred to as a "translation reaction promotion sequence"). Herein, the "translation reaction promotion sequence" refers to a sequence capable of improving the efficiency of translation by a factor of 1.2 or more (preferably by a factor of 2 or more) when cell-free protein synthesis is carried out using a DNA template containing the translation reaction promotion sequence, as compared to a case where cell-free protein synthesis is carried out using a DNA template not containing the translation reaction promotion sequence.

Such a translation reaction promotion sequence is not particularly limited, and a well-known sequence can be suitably used as long as it has the activity to promote translation reaction as described above. Specific examples of such a translation reaction promotion sequence include nucleotide sequences well known as 5' untranslated regions (hereinafter, simply referred to as "5' UTR") in *Bombyx mori* L. and baculovirus, such as a nucleotide sequence in the 5' UTR of the fibroin L-chain gene of *Bombyx mori* L., a nucleotide sequence in the 5' UTR of the sericin gene of *Bombyx mori* L., a nucleotide sequence in the 5' UTR of the polyhedrin gene of AcNPV *(Autographa californica* nuclear polyhedrosis virus), a nucleotide sequence in the 5' UTR of the polyhedrin gene of BmCPV *(Bombyx mori* cytoplasmin polyhedrosis virus), a nucleotide sequence in the 5' UTR of the polyhedrin gene of EsCPV *(Euxoa scandes* cytoplasmin polyhedrosis virus), a nucleotide sequence in the 5' UTR of the polyhedrin gene of HcNPV (*Hyphantria cunea* nuclear polyhedrosis virus), a nucleotide sequence in the 5' UTR of the polyhedrin gene of CrNPV *(Choristoneura rosaceana* nucleopolyhedrovirus), a nucleotide sequence in the 5' UTR of the polyhedrin gene of EoNPV *(Ecotropis oblique* nuclear polyhedrosis virus), a nucleotide sequence in the 5' UTR of the polyhedrin gene of MnNPV (*Malacosma neustria* nucleopolyhedrovirus), a nucleotide sequence in the 5' UTR of the polyhedrin gene of SfNPV (*Spodoptera frugiperda* nucleopolyhedrovirus), and a nucleotide sequence in the 5' UTR of the polyhedrin gene of WsNPV (*Wiseana signata* nucleopolyhedrovirus). These translation reaction promotion sequences can be obtained by any conventionally known method. For example, a translation reaction promotion sequence can be synthesized by using a well-known DNA synthesizer.

The DNA template preferably contains one or more translation reaction promotion sequences 5'upstream of the structural gene. The translation reaction promotion sequence(s) may be introduced in either a forward direction (5' → 3') or a reverse direction (3' → 5'), 5'upstream of the structural gene. In a case where two or more translation reaction promotion sequences are introduced into the DNA template, they are the same or different and all of them do not need to be introduced in the same direction. The translation reaction promotion sequence introduced 5'upstream of the structural gene may be adjacent to the structural gene, or may be located so that a nucleotide sequence containing one or more nucleotides is provided between the translation reaction promotion sequence and the structural gene.

The DNA template may further contain another sequence for a desired purpose in addition to the sequences described above. Specifically, in a case where a protein subjected to posttranslational modification is intended to be purified for subsequent experiment or the like, an appropriate sequence may be introduced into the DNA template. For example, in some case, the protein is preferably purified to facilitate SDS treatment before electrophoresis. In a case where the protein is purified by using a nickel column and the like, His-tag is added to the DNA template.

The transcription template is preferably contained in the reaction liquid for transcription/translation system in a proportion of 0.1 µg/mL - 8000 µg/mL, more preferably in a proportion of 3 µg/mL - 600 µg/mL. If the transcription template content is less than 0.1 µg/mL or exceeds 8000 µg/mL, the rate of protein synthesis tends to be lower.

The arthropod-derived microsomal membranes are contained in the reaction liquid for transcription/translation system so that the A260 becomes 1 - 50 (A260 = 1 - 50), preferably 2 - 15 (A260 = 2 - 15), from the viewpoint of efficiency of posttranslational modification of protein. If the A260 is less than 1, posttranslational modification tends to be insufficiently carried out. If the A260 exceeds 50, protein synthesis itself tends to be significantly inhibited. Further, in the translation reaction liquid, the ratio of the mRNA concentration (µg/mL) to the mammal-derived microsomal membrane concentration (A260) is preferably 1 : 0 .1 - 5, more preferably 1 : 0.3 - 2.3, from the viewpoint of efficiency of posttranslational modification of protein.

Examples of the arthropod-derived microsomal membranes include those prepared in the same manner as in the case of the reaction liquid for translation system.

The RNA polymerase to be contained in the reaction liquid for transcription/translation system is appropriately selected according to a promoter sequence of the transcription template. For example, in a case where the transcription template has a T7 promoter sequence, a T7 RNA polymerase that can recognize the T7 promoter sequence is preferably used. In a case where the transcription template has an SP6 or T3 promoter sequence, an SP6 RNA polymerase or a T3 RNA polymerase is preferably used, respectively.

The RNA polymerase is preferably contained in the reaction liquid for transcription/translation system in a proportion of 0.01 U/µL - 100 U/µL, more preferably in a proportion of 0.1U/µL - 10 U/µL, from the viewpoint of the rate of mRNA synthesis and the rate of protein synthesis. If the RNA polymerase content is less than 0.01 U/µL, the amount of mRNA to be synthesized is decreased so that the rate of protein synthesis tends to be lower. On the other hand, if the RNA polymerase content exceeds 100 U/µL, protein synthesis reaction tends to be inhibited.

The ATP is preferably contained in the reaction liquid for transcription/translation system in a proportion of 0.01 mM - 10 mM, more preferably in a proportion of 0.1 mM - 5 mM, from the viewpoint of the rate of protein synthesis. If the ATP content is less than 0.01 mM or exceeds 10 mM, the rate of protein synthesis tends to be lower.

The GTP is preferably contained in the reaction liquid for transcription/translation system in a proportion of 0.01 mM - 10 mM, more preferably in a proportion of 0.1 mM - 5 mM, from the viewpoint of the rate of protein synthesis. If the GTP content is less than 0.01 mM or exceeds 10 mM, the rate of protein synthesis tends to be lower.

The cytidine 5'-triphosphate (hereinafter, also simply referred to as "CTP") is preferably contained in the reaction liquid for transcription/translation system in a proportion of 0.01 mM - 10 mM, more preferably in a proportion of 0.1 mM - 5 mM, from the viewpoint of the rate of protein synthesis. If the CTP content is less than 0.01 mM or exceeds 10 mM, the rate of protein synthesis tends to be lower.

The uridine 5'-triphosphate (hereinafter, also simply referred to as "UTP") is preferably contained in the reaction liquid for transcription/translation system in a proportion of 0.01 mM - 10 mM, more preferably in a proportion of 0.1 mM - 5 mM, from the viewpoint of the rate of protein synthesis. If the UTP content is less than 0.01 mM or exceeds 10 mM, the rate of protein synthesis tends to be lower.

The creatine phosphate is a component for continuous synthesis of protein, and is added to the reaction liquid for transcription/translation system for regeneration of ATP and GTP. The creatine phosphate is preferably contained in the reaction liquid for transcription/translation system in a proportion of 1 mM - 200 mM, more preferably in a proportion of 10 mM - 100 mM, from the viewpoint of the rate of protein synthesis. If the creatine phosphate content is less than 1 mM, it is difficult to regenerate sufficient amounts of ATP and GTP so that the rate of protein synthesis tends to be lower. On the other hand, if the creatine phosphate content exceeds 200 mM, it acts as an inhibitor so that the rate of protein synthesis tends to be lower.

The creatine kinase is a component for continuous synthesis of protein, and is added, together with creatine phosphate, to the reaction liquid for transcription/translation system for regeneration of ATP and GTP. The creatine kinase is preferably contained in the reaction liquid for transcription/translation system in a proportion of 1 µg/mL - 1000 µg/mL, more preferably in a proportion of 10 µg/mL - 500 µg/mL, from the viewpoint of the rate of protein synthesis. If the creatine kinase content is less than 1 µg/mL, it is difficult to regenerate sufficient amounts of ATP and GTP so that the rate of protein synthesis tends to be lower. On the other hand, if the creatine kinase content exceeds 1000 µg/mL, it acts as an inhibitor so that the rate of protein synthesis tends to be lower.

The amino acid component in the reaction liquid for transcription/translation system contains at least 20 kinds of amino acids, i.e. , valine, methionine, glutamic acid, alanine, leucine, phenylalanine, glycin, proline, isoleucine, tryptophan, asparagine, serine, threonine, histidine, aspartic acid, tyrosine, lysine, glutamine, cystine, and arginine. This amino acid includes radioisotope-labeled amino acid. If necessary, modified amino acid may be contained. The amino acid component generally contains almost the same amount of various kinds of amino acids.

The amino acid component is preferably contained in the reaction liquid for transcription/translation system in a proportion of 1 µM - 1000 µM, more preferably in a proportion of 10 µM - 500 µM, from the viewpoint of the rate of protein synthesis. If the amino acid content is less than 1 µM or exceeds 1000 µM, the rate of protein synthesis tends to be lower.

The tRNA in the reaction liquid for transcription/translation system contains almost the same amount of tRNAs corresponding to the above-mentioned 20 kinds of amino acids. The tRNA is preferably contained in the reaction liquid for transcription/translation system in a proportion of 1 µg/mL - 1000 µg/mL, more preferably in a proportion of 10 µg/mL - 500 µg/mL, from the viewpoint of the rate of protein synthesis. If the tRNA content is less than 1 µg/mL or exceeds 1000 µg/mL, the rate of protein synthesis tends to be lower.

It is preferred that the reaction liquid for transcription/translation system further contains potassium salt, magnesium salt, DTT, RNase inhibitor, spermidine, and buffer.

Examples of the potassium salt in the reaction liquid for transcription/translation system include various potassium salts as mentioned above as a component of the solution for extraction. Among them, potassium acetate is preferably used. The potassium salt is preferably contained in the reaction liquid for transcription/translation system in a proportion of 10 mM - 500 mM, more preferably in a proportion of 50 mM - 150 mM, from the same point of view as the potassium salt contained in the solution for extraction.

Examples of the magnesium salt in the reaction liquid for transcription/translation system include various magnesium salts as mentioned above as a component of the solution for extraction. Among them, magnesium acetate is preferably used. The magnesium salt is preferably contained in the reaction liquid for transcription/translation system in a proportion of 0.1 mM - 10 mM, more preferably in a proportion of 0.5 mM - 3 mM, from the same point of view as the magnesium salt contained in the solution for extraction.

The DTT in the reaction liquid for transcription/translation system is added for prevention of oxidation, and is preferably contained in the reaction liquid in a proportion of 0.1mM - 100 mM, more preferably in a proportion of 0.2 mM - 20 mM. If the DTT content is less than 0.1 mM or exceeds 100 mM, components essential for protein synthesis tend to be unstable.

The RNase inhibitor in the reaction liquid for transcription/translation system is added to prevent RNase, which is contained in the arthropod-derived extract liquid, from undesirably digesting mRNA and tRNA and inhibiting protein synthesis during synthesis reaction in transcription/translation system. The RNase inhibitor is preferably contained in the reaction liquid in a proportion of 0.1 U/µL - 100 U/µL, more preferably in a proportion of 1 U/µL - 10 U/µL. If the RNase inhibitor content is less than 0.1 U/µL, the degradation activity of the RNase tends to be insufficiently suppressed. If the RNase inhibitor content exceeds 100 U/µL, protein synthesis reaction tends to be inhibited.

The spermidine is added to promote elongation reaction during transcription, and is preferably contained in the reaction liquid for transcription/translation system in a proportion of 0.01 mM - 100 mM, more preferably in a proportion of 0.05 mM - 10 mM. If the spermidine content is less than 0.01 mM, the rate of mRNA synthesis is lowered and the amount of mRNA to be synthesized is decreased so that the rate of protein synthesis tends to be lower. On the other hand, if the spermidine content exceeds 100 mM, protein synthesis reaction tends to be inhibited.

Preferred examples of the buffer to be contained in the reaction liquid for transcription/translation system include those mentioned above with reference to the solution for extraction. Among them, HEPES-KOH (pH 6 - 8) is preferably used for the same reason as described above. The amount of the buffer contained in the reaction liquid for transcription/translation system is preferably 1 mM - 200 mM, more preferably 5 mM - 50 mM, from the same point of view as the buffer contained in the solution for extraction.

It is preferred that the reaction liquid for transcription/translation system further contains glycerol. By adding glycerol, it is possible to stabilize components essential for protein synthesis during synthesis reaction in transcription/translation system. In a case where glycerol is added, the amount of glycerol is generally 5 (v/v)% - 20 (v/v)%.

As described above, the reaction liquid for transcription/translation system preferably contains, in addition to 30 (v/v)% - 60 (v/v)% of the extract liquid, 3 µg/mL - 600 µg/mL of transcription template, mammal-derived microsomal membranes (A260 = 1 - 50 in reaction liquid for transcription/translation system, final concentration ratio of mRNA translation template) (µg/mL) to microsomal membranes (A260) = 1 : 0.1 - 0.5), 0.1 U/µL - 10 U/µL of RNA polymerase, 0.1 mM - 5 mM of ATP, 0.1 mM - 5 mM of GTP, 0.1 mM - 5 mM of CTP, 0.1 mM - 5 mM of UTP, 10 mM - 100 mM of creatine phosphate, 10 µg/mL - 500 µg/mL of creatine kinase, 10 µM - 500 µM of amino acid component, and 10 µg/mL - 500 µg/mL of tRNA. Preferably, the reaction liquid for transcription/translation system further contains 50 mM - 150 mM of potassium acetate, 0.5 mM - 3 mM of magnesium acetate, 0.2 mM - 20 mM of DTT, 1 U/µL - 10 U/µL of RNase inhibitor, 0.05 mM - 10 mM of spermidine, 5 mM - 50 mM of HEPES-KOH (pH 7.4), and 5 (v/v)% - 20 (v/v)% of glycerol.

As in the case of the synthesis reaction in translation system described above, cell-free protein synthesis reaction using the reaction liquid for transcription/translation system (hereinafter, simply referred to as "synthesis reaction in transcription/translation system") is carried out in, for example, a low temperature incubator conventionally known. The reaction temperature in the transcription step is generally in the range of 10 - 60°C, preferably in the range of 20 - 50°C. If the reaction temperature in the transcription step is lower than 10°C, the rate of transcription tends to be lower. On the other hand, if the reaction temperature in the transcription step exceeds 60°C, components essential for reaction tends to be denatured. The reaction temperature in the translation step is generally in the range of 10 - 40°C, preferably in the range of 20 - 30°C. If the reaction temperature in the translation step is lower than 10°C, the rate of protein synthesis tends to be lower. On the other hand, if the reaction temperature in the translation step exceeds 40°C, components essential for reaction tends to be denatured.

Particularly, the synthesis reaction in transcription/translation system is preferably carried out at 20 - 30°C suitable for both of the transcription and translation steps, from the viewpoint of successively carrying out the transcription and translation steps. The total reaction time of the transcription and translation steps is generally 1 - 72 hours, preferably 3 - 24 hours.

The kind of protein to be synthesized using the reaction liquid for translation system or the reaction liquid for transcription/translation system is not particularly limited. However, in consideration of the object of the present invention, proteins which can undergo posttranslational modification are preferred. The amount of a synthesized protein can be measured by enzyme activity assay, SDS-PAGE, immunoassay, or the like. Whether or not posttranslational modification has been properly carried out can be determined by subjecting a synthesized protein to SDS-PAGE and fluorography to check a change in molecular weight due to the presence or absence of microsomal membranes and sensitivity to protease treatment.

### EXAMPLES

Hereinafter, the present invention will be described in more detail with reference to examples, but the present invention is not limited to these examples.

### [Example 1]

### (1) Preparation of Plasmid

Among various posttranslational modifications, glycosylation (N-glycosylation) was taken as an example, and *in vitro* synthesis of a glycoprotein was tried using pro-TNF-GLC proved to be glycosylated (which is a mutant-type human tumor necrosis factor containing an N-glycosylation site artificially introduced into the mature region thereof). Fig. 1 shows a schematic view of a plasmid constructed. The nucleotide sequence of polyhedrin 5'-UTR to be used is shown in SEQ ID No: 1, and the entire nucleotide sequence of pro-TNF-GLC gene is shown in SEQ ID No: 2.

The plasmid was constructed in the following manner. Using a pT_{N}T vector (manufactured by Promega) as a template and PCR primers containing *Bam*HI sites at the ends thereof, whose nucleotide sequences are shown in SEQ ID No: 3 and SEQ ID No: 4, respectively, self-ligation was carried out after digestion with *Bam*HI to introduce the *Bam*HI site in front of an *Xho*I site in a multicloning site. Further, PCR was carried out using PCR primers whose nucleotide sequences are shown in SEQ ID No: 5 and SEQ ID No: 6, respectively, and self-ligation was carried out after digestion with *Hind*III to change a *Bam*HI site originally existing in the pT_{N}T vector and located just after a T7 terminator sequence to a *Hind*III site. Then, PCR was carried out to amplify the region from the *Bam*HI site to the T7 promoter, and an insert previously prepared by synthesizing sense and antisense strands of the polyhedrin 5'-UTR and subjecting them to annealing was ligated into the region to construct a modified pT_{N}T vector. The annealed insert was prepared in the following manner. The synthesized sense and anti-sense strands were 5' end-phosphorylated with T4 polynucleotide kinase (manufactured by TOYOBO). After the reaction, the sense strand and the anti-sense strand were mixed together, and were then subjected to heat treatment at 95°C for 5 minutes. The mixture was left standing until the temperature thereof was decreased to room temperature to carry out annealing. Thereafter, the mixture was purified by ethanol precipitation, dissolved in water, applied to SigmaSpin Post Reaction Purification Column (manufactured by SIGMA) to remove excessive ATP, and again purified by ethanol precipitation.

The obtained modified pT_{N}T vector was digested with *Bam*HI*-Eco*RI. Then, a DNA fragment of about 0.7 kb (pro-TNF-GLC cDNA) obtained by inserting pro-TNF-GLC cDNA into a *Bam*HI-*Eco*RI site of a pBluescript vector to obtain a plasmid and digesting the plasmid with *Bam*HI-*Eco*RI was ligated as an insert into the modified pT_{N}T vector to construct a plasmid-I for *in vitro* transcription of pro-TNF-GLC gene.

Further, a plasmid-II for *in vitro* transcription of pro-TNF-GLC gene was prepared by inserting a histidine tag downstream of the pro-TNF-GLC of the plasmid-I for transcription. Specifically, an insert obtained by synthesizing sense and anti-sense strands for adding a histidine-tag shown in SEQ ID No: 7 and subjecting them to annealing was ligated into the modified pT_{N}T vector digested with SmaI to construct a modified pT_{N}T vector (His-Tag). A DNA fragment of about 0. 7 Kb amplified using the plasmid-I for transcription as a template and PCR primers shown in SEQ ID Nos: 8 and 9 was ligated as an insert into the modified pT_{N}T vector (His-Tag) digested with *Bam*HI-*Eco*RI. In this way, a plasmid-II for transcription was constructed.

All the PCR reactions were carried out for 30 cycles of 96°C for 15 sec, 50°C for 30 sec, and 68°C for 5 min using KOD-plus (manufactured by TOYOBO) with a DNA template denatured at 96°C for 2 minutes.

PCR (25 cycles of 96°C for 10 sec, 50°C for 5 sec, and 60°C for 4 min) was carried out using 250 ng of the DNA of the constructed plasmid as a template and Big Dye Terminator Cycle Sequence FS Ready Reaction Kit (manufactured by ABI), and then the reaction liquid was applied to ABI PRISM 310 Genetic Analyzer (manufactured by ABI) to analyze the nucleotide sequence of the plasmid.

All the ligation samples were transformed into *E. coli* DH5α (manufactured by TOYOBO) after ligation with Ligation High (manufactured by TOYOBO). Plasmid was prepared from the transformed *E. coli* by an alkaline-SDS method to analyze the DNA nucleotide sequence thereof.

### (2) In Vitro Transcription Reaction and Purification of mRNA

The plasmid prepared in the above (1) was digested with *Hind*III, and was then purified by phenol-chloroform extraction and ethanol precipitation. Using 1 µg of the obtained DNA as a template and RiboMax Large Scale RNA Production System-T7 (manufactured by Promega), mRNA synthesis was carried out in a volume of 20 µL at 37°C for 4 hours. After completion of the reaction, 1 U of RQ1 RNase Free DNase (manufactured by Promega) was added, and the mixture was incubated at 37°C for 15 minutes to digest the DNA template. Protein was removed from the mixture by phenol-chloroform extraction, and then ethanol precipitation was carried out. The obtained precipitate was dissolved in 100 µL of sterilizedwater, purified with NICK Column (manufactured by Amersham Biosciences), and again subjected to ethanol precipitation. The obtained precipitate was dissolved in sterilized water so that the A260/A280 was finally 1.8 - 2.0 and the final RNA concentration was 2 mg/mL. The thus prepared mRNA was directly used for cell-free protein synthesis. Hereinafter, mRNA transcribed from the plasmid-I for transcription will be referred to as "mRNA-I", and mRNA transcribed from the plasmid-II for transcription will be referred to as "mRNA-II".

### (3) Method for Preparing Microsomal Membranes from Cultured Insect Cells (High Five and Sf21)

### (3-1) Culture of Cultured Insect Cells (High Five and Sf21)

2.1 × 10⁷ High Five cultured insect cells (manufactured by Invitrogen) were cultured in a culture flask (600 cm²) containing Express Five serum-free medium (manufactured by Invitrogen) supplemented with L-glutamine at 27°C for 6 days. As a result, the number of cells reached 1.0 × 10⁸ and the weight of wet cells was 1.2 g.

Sf21 cultured insect cells (manufactured by Invitrogen) were cultured in Sf-900II SFM serum-free medium (manufactured by Invitrogen) at 27°C. Then, 6.0 × 10⁵ Sf21 cells permilliliter of medium were subjected to suspension culture in 50 mL of the medium in a 125 mL Erlenmeyer flask at 130 rpm at 27°C for 5 days. As a result, the number of cells reached 1.0 × 10⁸ and the weight of wet cells was 3 g.

### (3-2) Method for Preparing Microsomal Membranes

Microsomal membranes were prepared from the High Five cultured insect cells and the Sf21 cultured insect cells by sucrose density gradient ultracentrifugation based on a method developed by Walter, P. and Blobel, G. (see Enzymol. 96, 84-93, 1983). Specifically, the cultured insect cells cultured in the above (3-1) were collected and washed once with a solution for extraction described below (by centrifugation at 700 xg, 4°C for 10 min), and then the washed cells were suspended in the solution for extraction at a ratio of 1 gram of the cultured cells per 4 mL.

| [Composition of Solution for Extraction from High Five Cells] | |
|---|---|
| 30 mM | HEPES-KOH (pH 7.9) |
| 100 mM | KOAc |
| 2 mM | Mg(OAc)₂ |
| 0.25 mM | EGTA |
| 250 mM | sucrose |
| 2 mM | DTT |
| 0.5 mM | PMSF |

| [Composition of Solution for Extraction from Sf21 Cells] | |
|---|---|
| 40 mM | HEPES-KOH (pH 7.9) |
| 100 mM | KOAc |
| 1.5 mM | Mg(OAc)₂ |
| 0.1 mM | EGTA |
| 250 mM | sucrose |
| 2 mM | DTT |
| 0.5 mM | PMSF |

The suspension was homogenized by 20 strokes in a tightly fitting Dounce homogenizer to disrupt the cells. Thereafter, the obtained homogenate was centrifuged at 1000 xg, 4°C for 10 minutes, and then cell debris was removed to collect supernatant. From the supernatant, a microsomal membrane fraction was separated by sucrose density gradient ultracentrifugation in the following manner. First, a solution for extraction containing 1.3 M sucrose was placed in a container for ultracentrifugation, and then the supernatant was layered on the solution for extraction so that a volume ratio (v/v) of the solution for extraction to the supernatant is 1 : 3. The obtained sample was ultracentrifuged at 140,000 xg, 4°C for 2.5 hours using an ultracentrifugal separator CP80MX and a swing rotor P40ST (manufactured by HITACHI). After the ultracentrifugation, supernatant was completely discarded. On the other hand, precipitate was gently suspended in the solution for extraction at a ratio of 1 gram of initial wet weight of cells per 100 µL, and the suspension was used as a microsomal membrane. The ratio A260/A280 of the microsomal membrane was 1.4 - 1.5, and the A260 was about 150.

### (4) Cell-Free Protein Synthesis

### (4-1) Cell-Free Protein Synthesis Using Extract Liquid for Cell-Free Protein Synthesis Containing Arthropod-Derived Extract

Cell-free protein synthesis was carried out using the mRNA-I of pro-TNF GLC prepared in the above (2), the microsomal membranes prepared in the above (3), and an extract liquid for cell-free protein synthesis containing an arthropod-derived extract prepared.

### (Preparation of Extract Liquid Derived from Bombyx mori L.)

Fifteen *Bombyx mori* L. larvae reached day 4 of the fifth instar larval stage were prepared, and 3.07 g of posterior silk gland was isolated from the larvae using scissors, tweezers, a scalpel, and a spatula. The posterior silk gland was mashed in a mortar frozen at -80°C, and was then subjected to extraction using a solution for extraction with the following composition.

| [Composition of Solution for Extraction] | |
|---|---|
| 20 mM | HEPES-KOH (pH 7.4) |
| 100 mM | potassium acetate |
| 2 mM | magnesium acetate |
| 2 mM | DTT |
| 0.5 mM | PMSF |

After the completion of extraction, the obtained liquid product was centrifuged at 30,000 xg, 4°C for 30 minutes using a centrifugal separator (himac CR20B3 manufactured by HITACHI KOKI).

After the centrifugation, only the supernatant was isolated, and was again centrifuged at 30,000 xg, 4°C for 10 minutes. After the centrifugation, only the supernatant was isolated. A desalting column PD-10 (manufactured by Amersham Biosciences) was equilibrated with a solution for extraction containing 20% glycerol, and the supernatant was fed to the column and eluted with the solution for extraction to carry out gel filtration.

The absorbance of each of the fractions of filtrate obtained by gel filtration was measured at 280 nm using a spectrophotometer (Ultrospec3300pro manufactured by Amersham Biosciences), and a fraction(s) having an absorbance at 280 nm of 10 or higher was (were) collected and used as an extract liquid for cell-free protein synthesis derived from the posterior silk gland of *Bomhyx mori* L. larvae in the fifth instar larval stage.

The protein concentration of the obtained extract liquid was measured using a BCA Protein assay Kit (manufactured by PIERCE) in the following manner. First, 0.1 mL of the sample was added to 2 mL of a reaction reagent, and they were reacted at 37°C for 30 minutes. Then, the absorbance of the reaction mixture was measured at 562 nm using a spectrophotometer (Ultrospec3300pro manufactured by Amersham Biosciences). A calibration curve was prepared using BSA as a control.

The amount of the posterior silk gland of *Bombyx mori* L. larvae contained in the extract liquid was 17.5 mg/mL in terms of protein concentration.

### (Preparation of Extract Liquid Derived from Cultured Insect Cell)

### (1) Extract Liquid Derived from High Five

2.1 × 10⁷ High Five cultured insect cells (manufactured by Invitrogen) were cultured in a culture flask (600 cm²) containing Express Five serum-free medium (manufactured by Invitrogen) supplemented with L-glutamine at 27°C for 6 days. As a result, the number of cells reached 1.0 ×10⁸ and the weight of wet cells was 1.21 g.

Then, the cultured insect cells were collected and washed (by centrifugation at 700 xg, 4°C for 10 min) 3 times with a solution for washing with the following composition.

| [Composition of Solution for Washing] | |
|---|---|
| 60 mM | HEPES-KOH (pH 7.9) |
| 200 mM | potassium acetate |
| 4 mM | magnesium acetate |
| 4 mM | DTT |

The washed cultured insect cells were suspended in 1 mL of a solution for extraction with the following composition.

| [Composition of Solution for Extraction] | |
|---|---|
| 40 mM | HEPES-KOH (pH 7.9) |
| 100 mM | potassium acetate |
| 2 mM | magnesium acetate |
| 2 mM | calcium chloride |
| 20 (v/v)% | glycerol |
| 1 mM | DTT |
| 1 mM | PMSF |

The suspension was rapidly frozen in liquid nitrogen. After the suspension was sufficiently frozen, it was thawed in an ice-water bath at about 4°C. After the suspension was completely thawed, it was subjected to a centrifugal separator (himac CR20B3 manufactured by HITACHI KOKI) at 30,000 xg, 4°C for 10 min to collect supernatant. Then, 1.5 mL of the supernatant was applied to a desalting column PD-10 (manufactured by Amersham Biosciences) equilibrated with a buffer solution for gel filtration with the following composition.

| [Composition of Buffer Solution for Gel Filtration] | |
|---|---|
| 40 mM | HEPES-KOH (pH 7.9) |
| 100 mM | potassium acetate |
| 2 mM | magnesium acetate |
| 1 mM | DTT |
| 1 mM | PMSF |

After the application of the supernatant to a desalting column, the supernatant was eluted with 4 mL of the buffer solution for gel filtration. Then, the absorbance of each of the fractions of filtrate obtained by gel filtrationwasmeasured at 280 nm using a spectrophotometer (Ultrospec3300pro manufactured by Amersham Biosciences), and a fraction(s) having an absorbance at 280 nm of 30 or higher was (were) collected and used as a cultured insect cell-derived extract liquid.

### (2) Extract Liquid Derived from Sf21

Sf21 insect cells (manufactured by Invitrogen) were cultured in Sf900-II serum free medium (manufactured by Invitrogen) at 27°C. 6.0 × 10⁵ Sf21 insect cells per milliliter of medium were subjected to suspension culture in 50 mL of the medium in a 125 mL Erlenmeyer flask at 130 rpm at 27°C for 5 days. As a result, the number of cells per milliliter of medium reached 1.0 × 10⁸ and the weight of wet cells was 3 g.

Then, the cultured insect cells were collected and washed (by centrifugation at 700 xg, 4°C for 10 min) 3 times with a solution for washing with the following composition. The washed insect cells were suspended in 3 mL of a solution for extraction with the following composition.

| [Composition of Solution for Washing] | |
|---|---|
| 40 mM | HEPES-KOH (pH 7.9) |
| 100 mM | potassium acetate |
| 2 mM | magnesium acetate |
| 2 mM | calcium chloride |
| 1 mM | DTT |

| [Composition of Solution for Extraction] | |
|---|---|
| 40 mM | HEPES-KOH (pH 7.9) |
| 100 mM | potassium acetate |
| 2 mM | magnesium acetate |
| 2 mM | calcium chloride |
| 20 (v/v)% | glycerol |
| 1 mM | DTT |
| 0.5 mM | PMSF |

The cell suspension was rapidly frozen in liquid nitrogen. After the suspension was sufficiently frozen, it was thawed in an ice-water bath at about 4°C. After the suspension was completely thawed, it was subjected to a centrifugal separator (himac CR20B3 manufactured by HITACHI KOKI) at 30,000 xg, 4°C for 10 min to collect supernatant 1A. Then, the collected supernatant 1A was further subjected to a centrifugal separator (himac CR20B3 manufactured by HITACHI KOKI) at 45,000 ×g, 4°C for 30 min to collect supernatant 1B. Then, 2.5 mL of the collected supernatant 1B was applied to a desalting column PD-10 (manufactured by Amersham Biosciences) equilibrated with a buffer solution for gel filtration with the following composition.

| [Composition of Buffer Solution for Gel Filtration] | |
|---|---|
| 40 mM | HEPES-KOH (pH 7.9) |
| 100 mM | potassium acetate |
| 2 mM | magnesium acetate |
| 1 mM | DTT |
| 0.5 mM | PMSF |

After the application of the supernatant 1B to a desalting column, the supernatant 1B was eluted with 3 mL of the buffer solution for gel filtration. Then, the absorbance of each of the fractions of filtrate obtained by gel filtration was measured at 280 nm using a spectrophotometer (Ultrospec3300pro manufactured by Amersham Biosciences), and a fraction(s) having an absorbance at 280 nm of 30 or higher was (were) collected and used as an insect cell-derived extract liquid.

Cell-free protein synthesis were carried out using the extract liquid derived from the posterior silk gland of *Bombyx mori* L., the extract liquid derived from High Five cultured insect cell, and the extract liquid derived from Sf21 cultured insect cell, respectively. It is to be noted that the arthropod-derived microsomal membranes prepared in the above (3) were added at various concentrations at the beginning of each of the cell-free protein synthesis reactions to glycosylate a protein in the presence of the microsomal membranes. In a reference example, canine pancreatic microsomal membranes (manufactured by Promega) were used instead of the arthropod-derived microsomal membranes.

Hereinafter, the compositions of various reaction liquids containing the various extract liquids are shown, but the concentration of each of the components is a final concentration in the reaction liquid unless otherwise specified.

### Synthesis Using Extract Liquid Derived from Posterior Silk Gland of Bombyx mori L.

### insect-derived microsomal membranes (A260 = 0 - 50 in 1 µL of reaction liquid)

50 (v/v)% extract liquid derived from arthropod (that is, from posterior silk gland of *Bombyx mori L.)*

| | |
|---|---|
| 160 µg/mL | mRNA |
| 30 mM | HEPES-KOH (pH 7.4) |
| 100 mM | potassium acetate |
| 1.5 mM | magnesium acetate |
| 0.5 mM | DTT |
| 10 (v/v)% | glycerol |
| 0.75 mM | ATP |
| 0.5 mM | GTP |
| 0.25 mM | EGTA |
| 25 mM | creatine phosphate |
| 200 µg/mL | creatine kinase |
| 100 µM | amino acid (20 kinds) |
| 2 U/µL | RNase inhibitor |
| 100 µg/mL | tRNA |

ATP, GTP, and amino acid (20 kinds) were purchased from SIGMA, RNase inhibitor was purchased from TAKARA SHUZO, and tRNA was purchased from Roche Diagnostics.

As a reaction device, a low temperature dry thermo-bath MG-1000 (manufactured by Tokyo Rikakikai) was used. A translation reaction was carried out using 25 µL of the reaction liquid at 25°C for 6 hours.

### Synthesis Using Extract Liquid Derived from Cultured Insect Cell

### (1) Synthesis Using Extract Liquid Derived from High Five insect-derived microsomal membranes (A260 = 0 - 50 in 1 µL of reaction liquid)

50 (v/v)% extract liquid derived from arthropod (that is, from High Five)

| | |
|---|---|
| 320 µg/mL | mRNA |
| 40 mM | HEPES-KOH (pH 7.9) |
| 100 mM | potassium acetate |
| 2 mM | magnesium acetate |
| 2 mM | DTT |
| 10 (v/v)% | glycerol |
| 0.5 mM | ATP |
| 0.25 mM | GTP |
| 20 mM | creatine phosphate |
| 200 µg/mL | creatine kinase |
| 80 µM | amino acid (20 kinds) |
| 0.25 mM | EGTA |
| 1 U/µL | RNase inhibitor |
| 200 µg/mL | tRNA |

ATP, GTP, and amino acid (20 kinds) were purchased from SIGMA, RNase inhibitor was purchased from TAKARA SHUZO, and tRNA was purchased from Roche Diagnostics.

As a reaction device, a low temperature dry thermo-bath MG-1000 (manufactured by Tokyo Rikakikai) was used. A translation reaction was carried out using 25 µL of the reaction liquid at 25°C for 8 hours.

### (2) Synthesis Using Extract Liquid Derived from Sf21 insect-derived microsomal membrane (A260 = 0 - 50 in 1 µL of reaction liquid)

50 (v/v)% extract liquid derived from arthropod (that is, from Sf21)

| | |
|---|---|
| 40 mM | HEPES-KOH (pH 7.9) |
| 100 mM | potassium acetate |
| 1.5 mM | magnesium acetate |
| 2 mM | DTT |
| 10 (v/v)% | glycerol |
| 0.25 mM | ATP |
| 0.1 mM | GTP |
| 20 mM | creatine phosphate |
| 200 µg/mL | creatine kinase |
| 80 µM | amino acid (20 kinds) |
| 0.1 mM | EGTA |
| 1 U/µL | RNase inhibitor |
| 200 µg/mL | tRNA |
| 320 µg/mL | exogenous mRNA (pro-TNF-GLC gene) |

ATP, GTP, and amino acid (20 kinds) were purchased from SIGMA, RNase inhibitor was purchased from TAKARA SHUZO, and tRNA was purchased from Roche Diagnostics.

As a reaction device, a low temperature dry thermo-bath MG-1000 was used. A translation reaction was carried out using 25 µL of the reaction liquid at 25°C for 6 hours.

### (4-2) Cell-Free Protein Synthesis Using Extract Liquid for Cell-Free Protein Synthesis Containing Mammal-Derived Extract

Cell-free protein synthesis were carried out using the mRNA-II of pro-TNF GLC prepared in the above (2), the microsomal membranes prepared in the above (3), and an extract liquid derived from rabbit reticulocyte (RRL, manufactured by Promega). It is to be noted that the arthropod-derivedmicrosomal membranes prepared in the above (3) were added at various concentrations at the beginning of each of the cell-free protein synthesis reactions to glycosylate a protein in the presence of the microsomal membranes.
microsomal membranes (A260 = 0 - 50 in reaction liquid for translation system)

| | | |
|---|---|---|
| 50 (v/v)% | extract liquid derived from rabbit reticulocyte | 17.5 µL |
| 2 mg/mL | mRNA | 2 µL |
| 40 U/µL | RNase inhibitor | 1 µL |
| 1 mM | amino acid (20 kinds) | 1 µL |

Amino acid (20 kinds) was purchased from SIGMA, and RNase inhibitor was purchased from TAKARA SHUZO.

As a reaction device, a low temperature dry thermo-bath MG-1000 (manufactured by Tokyo Rikakikai) was used. A translation reaction was carried out using 25 µL of the reaction liquid at 30°C for 90 minutes.

### (5) Deglycosylation of Translation Reaction Product Synthesized by Cell-Free Protein Synthesis Described in (4)

In order to determine that N-glycosylated proteins (pro-TNF GLC) synthesized in the above (4) were glycosylated glycoproteins, the N-glycosylated proteins were subjected to deglycosylation using an N-deglycosylation enzyme in the following manner. 1 µL of glycopeptidase F (manufactured by TAKARA) as an N-deglycosylation enzyme was added per 10 µL of the reaction liquid after translation reaction, and they were reacted at 25°C for 2 hours.

### (6) Detection of N-glycosylated Protein

An N-glycosylated protein was detected by Western blotting using an anti-TNF antibody (manufactured by R&D) and chemiluminescence using ECL-plus (manufactured by Amersham Biosciences). Specifically, in a case where the insect cell-derived extract liquid was used, an equal volume of x2 Sample Buffer Solution (manufactured by Wako) was added for SDS treatment to the reaction liquid after translation reaction and deglycosylation reaction, and the mixture was treated with heat at 95°C for 3 minutes. The obtained sample was subjected to SDS-PAGE. On the other hand, in a case where the extract liquid derived from rabbit reticulocyte was used, as a pre-treatment of SDS treatment, 2 µL of PMSF (40 mM) and 200 µL of RIPA buffer (50 mM Tris-HCl pH 7.5, 150 mM NaCl, 1% Nonidet P-40, 0.5% sodium deoxycholate, 0.1% SDS) were added per 10 µL of the reaction liquid, stirred using a rotator at 4°C for 1 hour for solubilization, and simply purified using His-MicroSpin Purification Module (manufactured by Amersham Biosciences). To the obtained sample, an equal volume of x2 Sample Buffer Solution (manufactured by Wako) was added, and they were treated with heat at 95°C for 3 minutes. This sample was subjected to SDS-PAGE. After the completion of electrophoresis, proteins were transferred to a PVDF membrane. The PVDF membrane with transferred proteins was blocked by gently shaking at room temperature in TTBS (20 mM Tris, 500 mM NaCl, 0.05% Tween-20, pH 7.5) containing 3% gelatin. After blocking, the membrane was washed by shaking in the TTBS for 10 minutes. The proteins were reacted by gently shaking the membrane in the TTBS containing anti-TNF antibody (anti-TNF antibody / TTBS is 1 / 2000) and 1% gelatin for 2 - 12 hours. The membrane was washed by shaking in TCBS (20 mM citrate, 500 mM NaCl, 0.05% Tween-20, pH 5.5) for 5 min × 2. The proteins were reacted by gently shaking the membrane for 2 hours in a liquid obtained by adding 33 µL of proteinG-Horseradish Peroxidase Conjugate (manufactured by Bio Rad) per 100 mL of the TCBS containing 1% gelatin. The membrane was washed by shaking in the TTBS for 10 minutes. Thereafter, the proteins were visualized by chemiluminescence using ECL-plus before exposure of the membrane to an X-ray film and development of the film.

### (7) Result of Western Blotting

Figs. 2 and 3 show the results of Western blotting carried out in such a manner described above.

Fig. 2 shows the result of Western blotting of proteins synthesized through translation reactions in the presence of microsomal membranes using the extract liquid derived from the posterior silk gland of *Bombyx mori* L. (hereinafter, also simply referred to as "BML"), the extract liquid derived from High Five cultured insect cell (hereinafter, also simply referred to as "HFL"), and the extract liquid derived from Sf21 cultured insect cell (hereinafter, also simply referred to as "Sf21L"), respectively. In Fig. 2, "CMM" represents canine pancreatic microsomal membranes (manufactured by Promega), "HFMM" represents microsomal membranes derived from High Five cultured insect cell, and "Sf21MM" represents microsomal membranes derived from Sf21 cultured insect cell, and each of them was added to different reaction liquids to carry out translation reactions in the presence of microsomal membranes. The amount of the microsomal membranes added to the reaction liquid was expressed in terms of A260.

As can be seen from Fig. 2, in all the cases where BML, FHL, or Sf21L was used, addition of HFMM or Sf21MM allowed more efficient N-glycosylation as compared to a case where CMM was added. Further, synthesized proteins were confirmed to be N-glycosylated proteins from the fact that the shift bands thereof were significantly reduced by digestion with glycopeptidase F.

These results indicate that addition of the insect-derived microsomal membranes to the insect-derived extract liquid makes it possible to efficiently carry out glycosylation of protein.

Fig. 3 shows the result of Western blotting of proteins synthesized through translation reactions carried out in the presence of microsomal membranes using the extract liquid derived from rabbit reticulocyte. In Fig. 3, "HFMM" represents microsomal membranes derived from High Five cultured insect cell and "Sf21MM" represents microsomal membranes derived from Sf21 cultured insect cell, and each of them was added to the reaction liquid to carry out translation reactions in the presence of the microsomal membranes. As can be seen from Fig. 3, addition of HFMM or Sf21MM allowed efficient N-glycosylation. Further, synthesized proteins were confirmed to be N-glycosylated proteins from the fact that the shift bands thereof were significantly reduced by digestion with glycopeptidase F.

These results indicate that addition of the insect-derived microsomal membrane to the extract liquid derived from rabbit reticulocyte also makes it possible to efficiently carry out glycosylation of protein.

It is to be noted that in <223> of free text in sequence listing for explanation of artificial sequence, SEQ ID No: 1 is a nucleotide sequence of 5'-UTR of an EoNPV (baculovirus) polyhedrin gene, SEQ ID No: 2 is a nucleotide sequence of pro-TNF GLC cDNA, SEQ ID No: 3 is a PCR primer, SEQ ID No: 4 is a PCR primer, SEQ ID No: 5 is a PCR primer, SEQ ID No: 6 is a PCR primer, SEQ ID No: 7 is DNA encoding His-Tag (histidine-tag), SEQ ID No: 8 is a PCR primer, and SEQ ID No: 9 is a PCR primer.

## Claims

1. A method for synthesizing a protein in a cell-free system using an extract liquid for cell-free protein synthesis, the method comprising translation reaction in the presence of arthropod-derived microsomal membranes.

2. The method according to claim 1, wherein in the translation reaction, the ratio of the concentration of mRNA (µg/mL) to the concentration of the arthropod-derived microsomal membranes (A260) is 1 : 0.1 - 5.

3. The method according to claim 2, wherein the ratio is 1 : 0.3 - 2.3.

4. The method according to claim 1, wherein the arthropod-derived microsomal membranes are extracted from insect tissue.

5. The method according to claim 4, wherein the insect tissue is a tissue of *Bombyx mori* L.

6. The method according to claim 5, wherein the tissue of *Bombyx mori* L. is a fat body.

7. The method according to claim 1, wherein the arthropod-derived microsomal membranes are extracted from cultured insect cells.

8. The method according to claim 7, wherein the cultured insect cells are derived from an ovum of *Trichoplusia ni* or from an ovary cell of *Spodoptera frugiperda.*

9. The method according to claim 1, wherein the extract liquid for cell-free protein synthesis comprises an arthropod-derived extract.

10. The method according to claim 9, wherein the arthropod-derived extract is extracted from insect tissue.

11. The method according to claim 10, wherein the insect tissue is a tissue of *Bombyx mori L.*

12. The method according to claim 11, wherein the tissue of *Bombyx mori* L. comprises at least a posterior silk gland of *Bombyx mori* L. larva.

13. The method according to claim 9, wherein the arthropod-derived extract is extracted from cultured insect cells.

14. The method according to claim 13, wherein the cultured insect cells are derived from an ovum of *Trichoplusia ni* or from an ovary cell of *Spodoptera frugiperda*.

15. The method according to claim 1, wherein the extract liquid for cell-free protein synthesis comprises an extract derived from wheat germ.

16. The method according to claim 1, wherein the extract liquid for cell-free protein synthesis comprises an extract derived from cultured mammalian cells.

17. The method according to claim 1, wherein the extract liquid for cell-free protein synthesis comprises an extract derived from rabbit reticulocyte.

18. The method according to claim 1, wherein the extract liquid for cell-free protein synthesis comprises an extract derived from *Escherichia coli.*

19. The method according to claim 1, wherein the extract liquid for cell-free protein synthesis comprises an extract derived from yeast.

20. A method for posttranslational modification of protein in cell-free protein synthesis using an extract liquid for cell-free protein synthesis, the method comprising translation reaction in the presence of arthropod-derived microsomal membranes.

21. The method according to claim 20, wherein in the translation reaction, the ratio of the concentration of mRNA (µg/mL) to the concentration of the arthropod-derived microsomal membranes (A260) is 1 : 0.1 - 5.

22. The method according to claim 21, wherein the ratio is 1 : 0.3 - 2.3.

23. The method according to claim 20, wherein the arthropod-derived microsomal membranes are extracted from insect tissue.

24. The method according to claim 23, wherein the insect tissue is a tissue of *Bombyx mori L.*

25. The method according to claim 24, wherein the tissue of *Bombyx mori* L. is a fat body.

26. The method according to claim 20, wherein the arthropod-derived microsomal membranes are extracted from cultured insect cells.

27. The method according to claim 26, wherein the cultured insect cells are derived from an ovum of *Trichoplusia ni* or from an ovary cell of *Spodoptera frugiperda.*

28. The method according to claim 20, wherein the extract liquid for cell-free protein synthesis comprises an arthropod-derived extract.

29. The method according to claim 28, wherein the arthropod-derived extract is extracted from insect tissue.

30. The method according to claim 29, wherein the insect tissue is a tissue of *Bombyx mori L.*

31. The method according to claim 30, wherein the tissue of *Bombyx mori* L. comprises at least a posterior silk gland of *Bombyx mori* L. larva.

32. The method according to claim 28, wherein the arthropod-derived extract is extracted from cultured insect cells.

33. The method according to claim 32, wherein the cultured insect cells are derived from an ovum of *Trichoplusia ni* or from an ovary cell of *Spodoptera frugiperda.*

34. The method according to claim 20, wherein the extract liquid for cell-free protein synthesis comprises an extract derived from wheat germ.

35. The method according to claim 20, wherein the extract liquid for cell-free protein synthesis comprises an extract derived from cultured mammalian cells.

36. The method according to claim 20, wherein the extract liquid for cell-free protein synthesis comprises an extract derived from rabbit reticulocyte.

37. The method according to claim 20, wherein the extract liquid for cell-free protein synthesis comprises an extract derived from *Escherichia coli.*

38. The method according to claim 20, wherein the extract liquid for cell-free protein synthesis comprises an extract derived from yeast.

39. The method according to claim 20, wherein the posttranslational modification of protein is N-glycosylation and/or signal sequence cleavage.

40. An N-glycosylated protein which is obtained by the protein synthesis method according to claim 1.

41. A protein having a cleaved signal sequence, which is obtained by the protein synthesis method according to claim 1.
